(19) Europäisches Patentamt
European Patent Office
Office européen des brevets

(11) EP 1 244 650 B1

(12) **FASCICULE DE BREVET EUROPEEN**

(45) Date de publication et mention de la délivrance du brevet: **25.06.2003 Bulletin 2003/26**

(21) Numéro de dépôt: **00985339.1**

(22) Date de dépôt: **27.11.2000**

(51) Int Cl.[7]: **C07D 403/12**, C07D 251/18, C07D 251/52, C07D 251/54, A61K 31/53, A61K 31/506, A61P 35/00

(86) Numéro de dépôt international: **PCT/FR00/03310**

(87) Numéro de publication internationale: **WO 01/040218 (07.06.2001 Gazette 2001/23)**

(54) **DERIVES ARYLAMINES ET LEUR APPLICATION COMME AGENT ANTITELOMERASE**

ARYLAMINDERIVATE UND IHRE VERWENDUNG ALS ANTITELOMERASEMITTEL

ARYLAMINE DERIVATIVES AND THEIR USE AS ANTI-TELOMERASE AGENT

(84) Etats contractants désignés:
**AT BE CH CY DE DK ES FI FR GB GR IE IT LI LU MC NL PT SE TR**
Etats d'extension désignés:
**LT LV RO SI**

(30) Priorité: **29.11.1999 FR 9915031**
**11.08.2000 FR 0010561**

(43) Date de publication de la demande: **02.10.2002 Bulletin 2002/40**

(73) Titulaire: **Aventis Pharma S.A.**
**92160 Antony (FR)**

(72) Inventeurs:
- **MAILLIET, Patrick**
**F-94120 Fontenay sous Bois (FR)**
- **RIOU, Jean-François**
**F-51100 Reims (FR)**
- **MERGNY, Jean-Louis**
**F-94800 Villejuif (FR)**
- **LAOUI, Abdelazize**
**Bridgewater, NJ 08807 (US)**
- **LAVELLE, François**
**F-75012 Paris (FR)**
- **PETITGENET, Odile**
**F-75014 Paris (FR)**

(74) Mandataire: **Le Pennec, Magali et al**
**Aventis Pharma S.A.,Direction Brevets**
**20 avenue Raymond Aron**
**92160 Antony Cédex (FR)**

(56) Documents cités:
**WO-A-93/20056**
**WO-A-99/40087**
**DE-A- 19 812 879**
**US-A- 5 770 613**

- **PATENT ABSTRACTS OF JAPAN vol. 1999, no. 08, 30 juin 1999 (1999-06-30) & JP 11 060573 A (NIPPON KAYAKU CO LTD), 2 mars 1999 (1999-03-02)**
- **ALFRED KREUTZBERGER ET AL.: "Synthese und spektroskopische Untersuchungen von Dianilinotriazinen" CHEMIKER ZEITUNG, vol. 114, no. 6, 1990, pages 208-210, XP002143260**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 244 650 B1

## Description

La présente invention est relative à la thérapie du cancer et concerne de nouveaux agent anticancéreux ayant un mécanisme d'action bien particulier. Elle concerne aussi de nouveaux composés chimiques ainsi que leur application thérapeutique chez l'homme.

La présente invention concerne l'utilisation de nouveaux composés chimiques non nucléotidiques qui interagissent avec des structures spécifiques de l'acide désoxyribonucléique (ADN). Ces nouveaux composés sont constitués d'un agent répartiteur lié à deux groupes aminoaromatiques. Ces nouveaux composés sont utiles dans le traitement des cancers et agissent en particulier en tant qu'agents inhibiteurs de la télomérase. Ils sont particulièrement utiles pour stabiliser l'ADN en structure G-quadruplexe (tétrades de guanines). L'application thérapeutique de l'inhibition de la télomérase via la stabilisation de ces G-quadruplexes est l'arrêt de la mitose cellulaire et la mort des cellules à division rapide telles que les cellules cancéreuses et éventuellement l'induction de la sénescence des cellules cancéreuses.

Les composés de la présente invention présentent l'avantage du point de vue thérapeutique de bloquer la télomérase. Du point de vue biologique, la télomérase permet l'ajout de séquences d'ADN répétées du type T T A G G G, dites séquences télomériques, à l'extrémité du télomère, lors de la division cellulaire. Par cette action la télomérase rend la cellule immortelle. En effet, en l'absence de cette activité enzymatique, la cellule perd à chaque division 100 à 150 bases, ce qui la rend rapidement senescente. Lors de l'apparition de cellules cancéreuses à division rapide, il est apparu que ces cellules présentaient des télomères maintenus à une longueur stable au cours de la division cellulaire. Dans ces cellules cancéreuses il est apparu que la télomérase était fortement activée et qu'elle permettait l'addition de motifs répétés de séquences télomériques à la fin du télomère et permettait donc la conservation de la longueur du télomère dans les cellules cancéreuses. Il est apparu depuis quelques temps que plus de 85 % des cellules cancéreuses présentaient des tests positifs à la présence de télomérase alors que les cellules somatiques ne présentent pas cette caractéristique.

Ainsi la télomérase est une cible très convoitée pour traiter les cellules cancéreuses. La première approche évidente pour bloquer la télomérase a été l'utilisation de structures nucléotidiques (Chen et al., Proc. Natl. Acad. Sci. USA 93(7), 2635-2639). Parmi les composés non nucléotidiques qui ont été utilisées dans l'art antérieur on peut citer les diaminoanthraquinones (Sun et al. J. Med. Chem. 40(14), 2113-6) ou les diethyloxadicarbocyanines (Wheelhouse R. T. Et al. J. Am. Chem. Soc. 1998(120) 3261-2).

Le brevet WO 99/40087 décrit l'utilisation de composés qui interagissent avec les structures G-quadruplexes qui sont des composés perylenes et des carbocyanines contenant au moins sept cycles dont deux heterocycles.

Il est apparu de façon tout à fait surprenante que des structures simples permettaient d'obtenir un résultat au moins équivalent avec des structures beaucoup moins compliquées du point de vue chimique. Les composés de la présente invention qui répondent à l'objectif visé c'est-à-dire qui fixent la structure G-quadruplex et par ce fait présentent une activité inhibitrice des télomérases répondent à la formule générale suivante :

cycle aromatique azoté- $NR_3$ - répartiteur-$NR'_3$ - cycle aromatique

dans laquelle

- le cycle aromatique azoté, représente :
  - ◊ une quinoléine éventuellement substituée par au moins
    - un groupe N(Ra)(Rb) dans lequel Ra et Rb, identiques ou différents représentent l'hydrogène ou un radical alkyle en C1-C4 ou
    - un groupe alkoxy à chaîne courte en C1-C4 ou
  - ◊ une quinoléine possédant un atome d'azote sous forme quaternaire ou
  - ◊ une benzamidine ou
  - ◊ une pyridine
- le cycle aromatique représente
  - ◊ une quinoléine éventuellement substituée par au moins un groupe N(Ra)(Rb) dans lequel Ra et Rb, identiques ou différents représentent l'hydrogène ou un radical alkyle et/ou alkoxy à chaîne courte en C1-C4 et/ou

◊ une quinoléine possédant un atome d'azote sous forme quaternaire ou
◊ une benzamidine ou
◊ une pyridine ou
◊ un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4 ou
◊ un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4

- R3 et R'3, identiques ou différents, représentent indépendamment l'un de l'autre l'hydrogène ou un radical alkyle en C1-C4
- le répartiteur représente :

◊ un groupe triazine éventuellement substitué par un radical alkyle ayant 1 à 4 atomes de carbone, un radical thio, oxy ou amino eux même éventuellement substitués par un ou plusieurs chaines alkyle à chaine courte contenant 1 à 4 atomes de carbone ou un atome d'halogène ou

◊ un groupe carbonyle ou

◊ un groupe C(=NH)-NH-C(=NH) ou

◊ un groupe alkylediyle contenant 3 à 7 atomes de carbone ou

◊ un groupe diazine éventuellement substitué par les mêmes groupes que la triazine

ou un de ses sels.

On entend au sens de la formule ci-dessus par cycle aromatique azoté un hétérocycle comportant au moins un atome d'azote ou un groupe aromatique ne comportant pas d'hétéroatome dans le cycle mais contenant au moins un atome d'azote dans une chaîne hydrocarbonée liée au cycle comme par exemple une chaîne guanidino ou guanyl.

On préfère parmi l'ensemble des composés ci-dessus inclus utiliser ceux comportant comme répartiteur un groupe triazine ou diazine. Parmi les groupes diazines on préfère utiliser les pyrimidines. Parmi les triazines on préfère les composés répondant à la formule (1) ci-dessous :

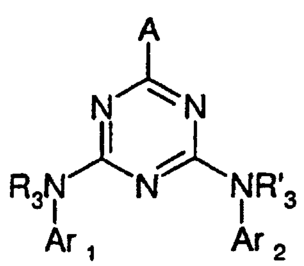

dans laquelle :

- A représente

  - un groupe amino de formule NR1R2 dans lequel R1 et R2 identiques ou différents représentent l'hydrogène ou un groupe alkyle droit ou ramifié contenant 1 à 4 atomes de carbone ou

  - un groupe OR1 ou SR1 dans lequel R1 a la même signification que précédemment ou

  - un groupe alkyle contenant 1 à 4 atomes de carbone ou ou un groupe trifluorométhyle ou

  - un atome d'hydrogène ou

- un atome d'halogène choisi parmi le fluor, le chlore, le brome ou l'iode

- R3 et R'3, identiques ou différents, représentent indépendamment l'un de l'autre l'hydrogène ou un radical alkyle en C1-C4.
- $Ar_1$ et $Ar_2$ identiques ou différents représentent

  1. quand $Ar_1$ et $Ar_2$sont identiques :

  - un motif quinoléine éventuellement substitué par au moins

    - un groupe N(Ra)(Rb) dans lequel Ra et Rb identiques ou différents représentent l'hydrogène ou un radical alkyle en C1-C4 ou
    - un groupe alkoxy à chaîne courte contenant 1 à 4 atome de carbone ou

  - une quinoléine possédant un atome d'azote sous forme quaternaire ou
  - une benzamidine ou
  - une pyridine attachée en position -4 ou fusionnée avec un groupe aryle ou hétéroaryle éventuellement substituée par un groupe alkyle en C1-C4

  2. quand $Ar_1$ et $Ar_2$ sont différents

  - $Ar_1$ et $Ar_2$ représentent tous les deux l'une des possibilités évoquées ci-dessus pour $Ar_1$ et $Ar_2$ ou

  - $Ar_1$ représente l'une des possibilités ci-dessus et $Ar_2$ représente

    * un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4

    * un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4

ou un de ses sels.

Il est évident que les motifs quinoléines peuvent être substitués par tout autre groupe n'intervenant pas dans l'application visée, ainsi des groupes acridines ou isoquinoléines ou quinazolines ou quinoxalines ou phtalazines ou benzothiazines ou benzoxazines ou phénoxazines ou phénothiazines sont inclus dans la définition des groupes quinoléines.

On préfère parmi les composés de formule (I) ci-dessus ceux qui comportent deux hétérocycles choisis parmi les groupes 4-aminoquinolyl, 4-aminoquinolinium ou quinolinium dont le noyau quinolinium est éventuellement substitué par un groupe méthyle.

En ce qui concerne les groupes A, ils représentent de préférence le radical méthylthio, amino, alkylamino ou dialkylamino radicaux dans lesquels les groupes alkyle possèdent 1 à 4 atomes de carbone.

On peut citer à titre de composés représentatifs de la formule (I) les composés suivants:

- le dichlorure de 2-amino-bis-4,6-[(1'-méthyl-4'-amino-6'-quinaldinio) amino]-triazine
- le dichlorure de 2-amino-bis-4,6-[(1'-éthyl-4'-amino-6'-quinaldinio) amino]-triazine
- le dichlorure de 2-diméthylamino-bis-4,6-[(1'-méthyl-4'-amino-6'-quinaidinio)amino]-triazine
- le trichlorhydrate de 2-méthylamino-bis-4,6-[(4'-amino-6'-quinaldinyl)amino]-triazine
- le dichlorure de 2-amino-bis-4,6-[(1'-méthyl-6'-quinoléinio)amino]-triazine
- le trichlorhydrate du dichlorure de 2-méthylamino-bis-4,6-[(4'méthylamino-6'-quinaldinyl)amino]-triazine
- le chlorhydrate du dichlorure de 2-amino-bis-4,6-[(9'-amino-10'-méthyl-2'-acridinio)amino]-triazine
- le trichlorhydrate de 2-amino-bis-4,6-[(4'-amino-6'-quinaldinyl) amino]-triazine
- le trichlorhydrate de 2-amino-bis-4,6-(p-amidinoanilino)-triazine,
- le dichlorure de 2-méthylthio-bis-4,6-[(1'-méthyl-4'-amino-6'-quinaldinio) amino]-triazine
- le dichlorhydrate dihydrate de 2-chloro-bis-4,6-[(4'-diméthylamino-6'-quinaldinyl)amino]-triazine

- l'hydrate de 2-méthylthio-bis-4,6-[(4'-diméthylamino-6'-quinaldinyl) amino]-triazine
- le dichlorhydrate de N,N'-(4-amino-6-quinaldinyl)urée
- le diodure de $N^1,N^5$-bis(7-chloro-1-méthyl-4-quinoléinio)pentane-1,5-diamine
- le trichlorhydrate pentahydrate de bis-2,4-[(4'-amino-6'-quinaldinyl) amino]pyrimidine
- le trichlorhydrate dihydrate de 1,5-(4'-amino-6'quinaldinyl)biguanide
- le 6-[4-(4-amino-2-méthyl-quinolin-6-ylamlno)-6-méthylsulfanyl-[1,3,5]triazin-2-ylamino]-2-méthyl-quinolin-4-ol
- la N6-[4-(4-diméthylamino-2-méthyl-quinolin-6-ylamino)-6-méthyl-sulfanyl-[1,3,5]triazin-2-yl]-2-méthyl-quinolin-4,6-diamine
- la N6-[4-(4-amino-2-méthyl-quinolin-6-y(amino)-6-méthylsulfanyl-[1,3,5]triazin-2-yl]-2-méthyl-quinolin-4,6-diamine
- la N6-[4-(4-méthoxy-2-méthyl-quinolin-6-ylamino)-6-méthylsulfanyl-[1,3,5]triazin-2-yl]-4-méthoxy-2-méthyl-quinolin-6-amine
- la N6-[(6-(4-amino-quinaldin-6-yl-amino)-4-méthylthio-triazin-2-yl]-quinaldine-4,6-diamine
- la N6-[(6-(4-diméthylamino-quinaldin-6-yl-amino)-4-méthylthiotriazin-2-yl]-quinaldine-4,6-diamine
- la N6-[(6-(quinolyl-6-yl-amino)-4-diethylamino-triazin-2-yl]-quinaldine-4,6-diamine

On préfère tout particulièrement l'hydrate de 2-méthylthio-bis-4,6-[(4'-diméthylamino-6'-quiraaldinyl) amino]-triazine.

Un autre objet de la présente invention concerne les composés de formule (I) en tant que produits chimiques nouveaux. Il concerne donc les produits nouveaux répondant à la formule (I) suivante :

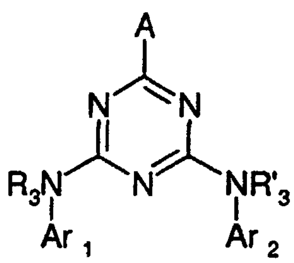

dans laquelle :

- A représente
  - un groupe amino de formule NR1R2 dans lequel R1 et R2 identiques ou différents représentent un groupe alkyle droit ou ramifié contenant 1 à 4 atomes de carbone ou
  - un groupe OR1 ou SR1 dans lequel R1 représente l'hydrogène ou a la même signification que précédemment ou
  - un groupe alkyle contenant 1 à 4 atomes de carbone ou un groupe trifluorométhyle ou
  - un atome d'hydrogène ou
  - un atome d'halogène choisi parmi le fluor, le chlore, le brome ou l'iode

- $R_3$ et $R'_3$, identiques ou différents, représentent indépendamment l'un de l'autre un atome d'hydrogène ou un groupe alkyle en C1-C4
- $Ar_1$ et $Ar_2$ identiques ou différents représentent

  1.quand $Ar_1$ et $Ar_2$ sont identiques :

  - un motif quinoléine éventuellement substitué par au moins
    - un groupe N(Ra)(Rb) dans lequel Ra et Rb identiques ou différents représentent l'hydrogène ou un radical alkyle en C1-C4 ou
    - un groupe alkoxy à chaîne courte contenant 1 à 4 atome de carbone ou
  - une quinoléine possédant un atome d'azote sous forme quaternaire ou

- une benzamidine sauf dans le cas où A représente la diéthylamine, l'hydrogène ou un groupe amine
- une pyridine attachée en position -4 ou fusionnée avec un groupe aryle ou hétéroaryle éventuellement substituée par un groupe alkyle en C1-C4

2. quand $Ar_1$ et $Ar_2$ sont différents

- $Ar_1$ et $Ar_2$ représentent tous les deux l'une des possibilités évoquées ci-dessus pour $Ar_1$ et $Ar_2$ ou
- $Ar_1$ représente l'une des possibilités ci-dessus et $Ar_2$ représente

  * un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4
  * un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4

ou un de ses sels à l'exclusion du dichlorhydrate de la 2-amino-bis-4,6-[(4'-amino-6'-quinaldinyl)amino]-triazine et de la 2-amino-bis-4,6-(p-amidinoanilino)-triazine.

En effet le premier de ces deux composés est décrit dans une publication parue sous la référence Indian Journal of Animal Sciences 43(4), pages 226-29 comme agent antitrypanosome pour l'animal et en aucun cas comme agent antitélomérase et le second est aussi décrit comme agent antitrypanosome dans J. Chem. Soc., 1960, 4525

Les composés de formule (I) qui sont préférés sont ceux pour lesquels $Ar_1$ et $Ar_2$ représentent un groupe choisi parmi les motifs suivants : 4-amino- ou 4-méthylamino- ou 4-diméthylamino-quinolyl ou quinolinium dont le noyau quinolinium est éventuellement substitué par un groupe méthyle.

Les composés de formule générale (I) qui sont préférés sont ceux pour lesquels A représente un groupe amino ou diméthylamino ou plus préférentiellement méthylthio.

On préfère tout particulièrement les composés de formule (I) pour lesquels lorsque $Ar_1$ et $Ar_2$ sont différents :

1. $Ar_1$ représente :

- un motif quinoléine substitué par au moins
  - un groupe N(Ra)(Rb) dans lequel Ra et Rb identiques ou différents représentent l'hydrogène ou un radical alkyle en C1-C4 ou
  - un groupe alkoxy à chaîne courte contenant 1 à 4 atome de carbone ou
- une quinoléine possédant un atome d'azote sous forme quaternaire ou
- une benzamidine sauf dans le cas où A représente la diéthylamine, l'hydrogène ou un groupe amine ou
- une pyridine attachée en position -4 ou fusionnée avec un groupe aryle ou hétéroaryle

2. $Ar_2$ représente

* un noyau tel que défini ci-dessus mais différent ou
* un noyau phényle éventuellement substitué par un groupement halogène, méthoxy, cyano, carbonylamino, guanyl, méthylthio, amino, méthylamino, diméthylamino, morpholine, alkylèneamino en C1-C4 ou alkénylèneamino en C2-C4
* un noyau quinoline, benzimidazole, indole, benzothiophène, benzofurane, benzothiazole, benzoxazole, carbazole, quinazoline, quinoxaline éventuellement substitué par un ou plusieurs groupes alkyle en C1-C4 ou

par des groupes alkylène en C1-C4 ou alkénylène en C2-C4

ou un de ses sels à l'exclusion du dichlorhydrate de la 2-amino-bis-4,6-[(4'-amino-6'-quinaldinyl)amino]-triazine et de la 2-amino-bis-4,6-(p-amidinoanilino)-triazine.

Un autre objet de la présente invention concerne l'utilisation des composés de la formule (I) comme produit pharmaceutique à usage humain.

Les procédés de préparation des composés de formule (I)

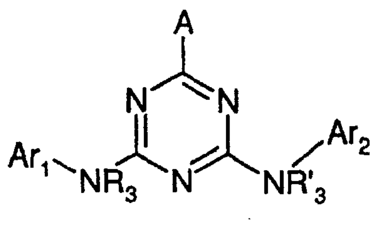
(I)

sont décrits ci-après.

Méthode générale 1

Selon un premier procédé de préparation des composés de formule générale (I) dans lesquels $Ar_1$ et $Ar_2$ d'une part et $R_3$ et $R'_3$ d'autre part sont identiques et définis tels que précédemment et R représente un atome d'halogène tel que chlore ou fluor, une fonction amino, alkylamino ou dialkylamino dont les parties alkyles droites ou ramifiées contiennent de 1 à 4 atomes de carbone, alkyloxy ou alkylthio dont les parties alkyles droites ou ramifiées contiennent de 1 à 4 atomes de carbone, peuvent être obtenus par amination d'une dihalogénotriazine, très généralement une dichloro-striazine, de formule générale (B) dans laquelle A est défini comme ci-dessus par une amine aromatique ou hétéroaromatique de formule générale (C) dans laquelle Ar est défini comme précédemment en opérant selon le schéma 1 :

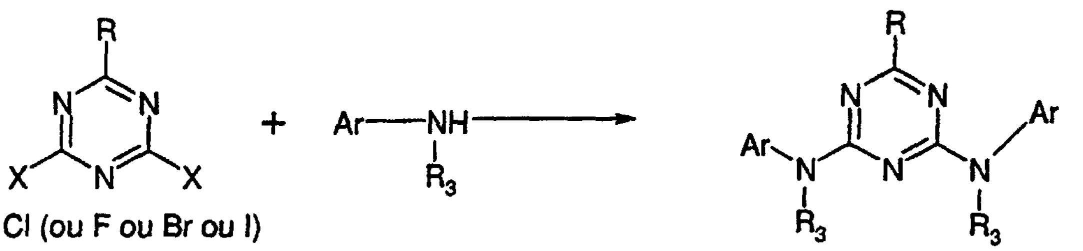
X = Cl (ou F ou Br ou I)

(B) (C) (A)

Schéma 1

Dans le cas où A représente un atome d'halogène, il est utile de faire réagir la 2,4,6-trihalogéno-s-triazine correspondante de formule générale (B) avec l'amine aromatique ou hétéroaromatique $ArNHR_3$ de formule générale (C).

On opère généralement en condensant une mole de dihalogéno-s-triazine, ou trihalogéno-s-triazine, avec 2 moles d'amine aromatique ou hétéroaromatique. La réaction a lieu en milieu inerte dans les conditions de la réaction. On peut citer parmi les solvants inertes l'acétone éventuellement aqueux ou un alcool éventuellement aqueux comme l'éthanol, ou un solvant halogéné tel que le dichlorométhane, ou un éther tel que l'oxyde de diéthyle ou le dioxane, ou un solvant aprotique polaire tel que le DMF le DMSO ou la NMP. On opère de préférence à une température comprise entre 20°C et le reflux, en présence notamment d'une base organique, telle que la triéthylamine, ou minérale, telle que la soude ou le carbonate de sodium ou de potassium . Il est également possible de ne pas utiliser de base lors de la réaction domination, et d'isoler un chlorhydrate du produit de formule générale (A), dont la base peut ensuite être libérée.

Les dihalogéno ou trihalogéno-s-triazines de formule générale (B) sont soit commerciales soit connues, et

peuvent être obtenues dans les conditions décrites dans la littérature.

Les amines aromatiques ou hétéroaromatiques de formule générale (C) sont soit connues, soit peuvent être préparées aisément par les méthodes connues de synthèse d'aminés aromatiques ou hétéroaromatiques.

Dans le cas où $Ar_1$ et $Ar_2$ sont différents, la triazine de formule générale (A) peut être obtenue par déplacement séquentiel des atomes d'halogène, très généralement des atomes de chlore, des produits de formule générale (B) par les aminés $Ar_1NHR_3$ puis $Ar_2NHR'_3$ de formule générale (C) selon le schéma 2 :

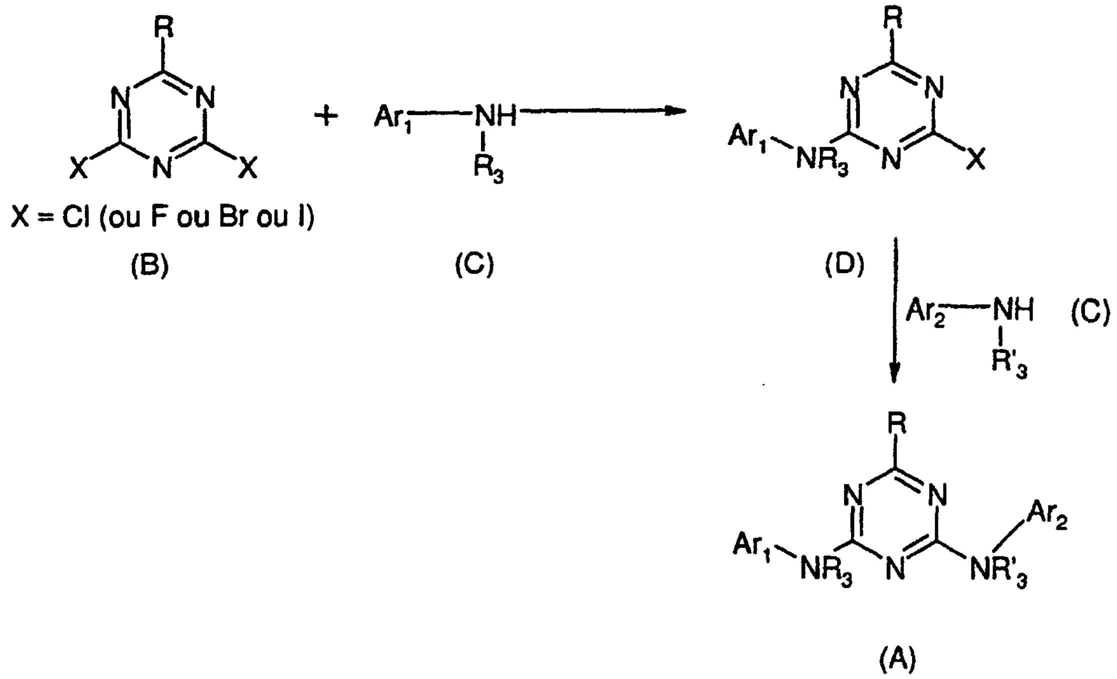


(B) (C) (D) (C) (A)

Schéma 2

Généralement on opère avec 1 mole de dihalogéno-s-triazine, ou trihalogéno-s-triazine, et 1 mole d'amine $Ar_1NHR_3$. On préfère opérer dans un solvant inerte tel que l'acétone éventuellement aqueux ou un alcool éventuellement aqueux, comme l'éthanol, ou un solvant halogéné, tel que le dichlorométhane, ou un éther tel que l'oxyde de diéthyle ou le dioxane, ou un solvant aprotique polaire tel que le DMF le DMSO ou la NMP. Selon une meilleure manière de mettre en oeuvre l'invention on opère à une température comprise entre 20°C et 50°C. Ensuite on ajoute 1 mole d'amine $Ar_2NHR'_3$ au produit de formule générale (D), qui peut être éventuellement isolé. On opère notamment à une température comprise entre 50°C et le reflux.

Avantageusement, on peut opérer dans les conditions décrites dans J. Fluor. Chem., 1988, 39(1), 117-123.

Méthode générale 2

Selon une seconde méthode les produits de formule générale (A) dans lesquels $Ar_1NHR_3$ et $Ar_2NHR'_3$ sont définis tels que précédemment et R représente un groupe NR1R2 ou OR1 ou SR1 peuvent être également préparés par déplacement nucléophile d'un atome d'halogène, généralement un atome de chlore, d'un produit de formule générale (A) dans lequel R représente un atome d'halogène selon le schéma 3 :

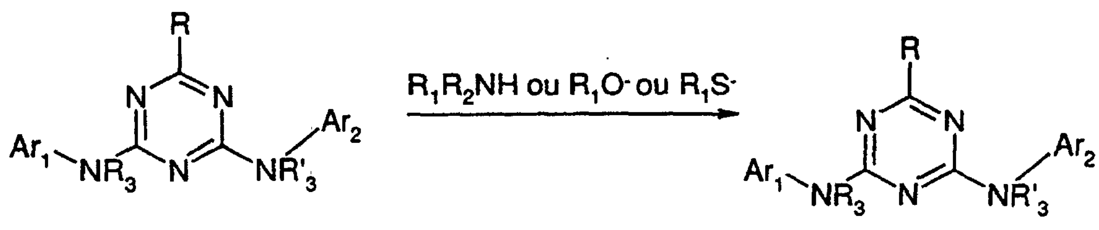


(A) (A)

R = Cl (ou F ou Br ou I) R= $NR_1R_2$ ou $OR_1$ ou $SR_1$

Schéma 3

On opère généralement en condensant 1 mole de produit de formule générale (A) dans lequel R représente un atome d'halogène, préférentiellement un atome de chlore, avec 1 mole d'amine R1R2NH ou d'alcoolate R1O$^-$ ou de thioalcoolate R1S. La réaction a lieu en milieu inerte dans les conditions de la réaction. On peut citer parmi les solvants inertes l'acétone éventuellement aqueux ou un alcool éventuellement aqueux comme l'éthanol, ou un solvant halogéné tel que le dichlorométhane, ou un éther tel que l'oxydé de diéthyle ou le dioxane, ou un solvant aprotique polaire tel que le DMF le DMSO ou la NMP. Lorsque le groupe entrant représente un groupé R1R2NH, on opère de préférence à une température comprise entre 20°C et le reflux, en présence notamment d'une base organique, telle que la triéthylamine, ou minérale, telle que la soude ou le carbonate de sodium ou de potassium. Il est également possible de ne pas utiliser de base lors de la réaction d'amination, et d'isoler un chlorhydrate du produit de formule générale (A), dont la base peut ensuite être libérée. Lorsque le groupe entrant représente un groupe R1O$^-$ ou R1S$^-$ on opère préférentiellement avec un alcoolate ou un thioalcoolate alcalin ou alcalinoterreux, tel qu'un sel de sodium ou de potassium ou de lithium ou d'ammonium ou de césium ou de baryum, dans un solvant aprotique polaire tel que le DMF ou le DMSO ou la NMP, à une température comprise entre 50°C et le reflux.

Méthode générale 3

Selon un troisième procédé de préparation les composés pour lesquels R représente un atome d'hydrogène ou un groupe alkyle, droit ou ramifié contenant de 1 à 4 atomes de carbone, peuvent également être préparés par condensation d'un bisguanide de formule générale (E), dans lequel $Ar_1$ et $Ar_2$ d'une part et $R_3$ et $R'_3$ d'autre part sont identiques ou différents, avec un dérivé d'acide, préférentiellement un chlorure d'acide ou un ester de méthyle de formule générale (F) selon le schéma 4 :

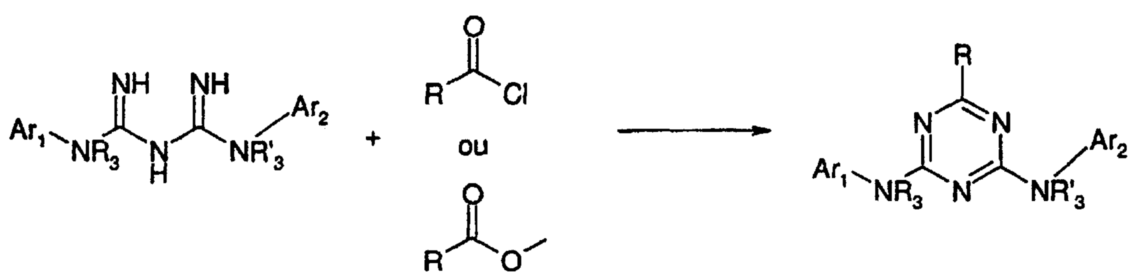


(E) (F) (A)

Schéma 4

La condensation entre le bisguanide de formule générale (E) et le dérivé d'acide de formule générale (F) est

effectuée généralement dans un alcool comme le méthanol ou l'éthanol. On préfère opérer à une température comprise entre 0°C et la température de reflux.

Les bisguanides de formule générale (E) symétriques ou dissymétriques peuvent être obtenus en opérant dans les conditions décrites dans la littérature et en particulier selon le brevet J.P. 94-4993.

Méthode générale 4

Les produits de formule générale (A), dans lesquels $Ar_1$ et $Ar_2$ sont identiques, définis comme précédemment et représentés par Ar, et où R représente un groupe alkyle, droit ou ramifié contenant de 1 à 4 atomes de carbone, peuvent également être préparés par condensation d'une cyanoguanidine de formule générale (G), dans laquelle Ar est défini comme précédemment, avec un nitrile de formule générale (H) selon le schéma 4 :

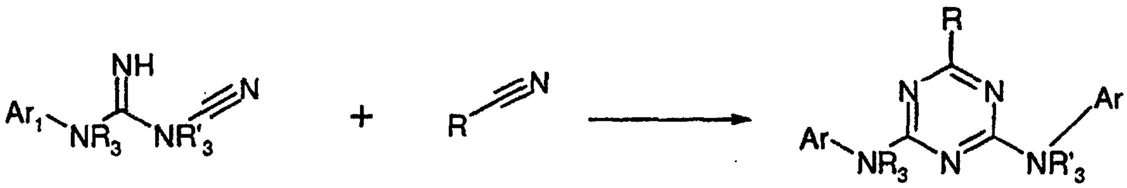

(G) (H) (A)

Schéma 5

La condensation de la cyanoguanidine de formule générale (G) avec le nitrile de formule générale (H) est notamment effectuée en opérant au reflux d'un solvant polaire à haut point d'ébullition tel que le 2-méthoxyéthanol ou le 1,2-diméthoxyéthane.

Les cyanoguanidines de formule générale (G) peuvent être préparées dans les conditions décrites dans la littérature.

Il est entendu que les s-triazines de formule générale peuvent être obtenues sous forme de librairies, en appliquant les méthodes décrites dans les schémas 1, 2, 3, 4 ou 5 en chimie parallèle et/ou combinatoire en phase liquide ou en phase solide, étant entendu que, lorsqu'on travaille en phase solide, l'un quelconque des réactifs est préalablement fixé sur un support solide, choisi en fonction de la réaction chimique mise en jeu, et que ladite réaction chimique est suivie d'une opération de clivage du produit de la réaction du support solide.

La présente invention concerne aussi les compositions thérapeutiques contenant un composé selon l'invention, en association avec un support pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer

- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine

- les dérivés du platine comme notamment le cisplatine, le carboplatine ou l'oxaliplatine

- les agents antibiotiques comme notamment la bléomycine, la mitomycine, la dactinomycine,

- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les topoisomérases des groupes I et II telles.que l'étoposide, le teniposide, l'amsacrine, l'irinotecan, le topotecan et le tomudex,
- les fluoropyrimidines telles que le 5-fluorouracile, l'UFT, la floxuridine,
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oetrogéniques, androgéniques
- les agents antivasculaires tels que les dérivés de la combretastatine ou de la colchicine et leur prodrug.

Il est également possible d'associer aux composés de la présente invention un traitement par les radiations. Ces traitements peuvent être administrés simultanément, séparément, séquentiellement. Le traitement sera adapté au malade à traiter par le praticien.

L'activité de stabilisation des G-quadruplexes peut être déterminée par une méthode utilisant la formation d'un complexe avec la fluoresceine dont le protocole expérimental est décrit ci-après.

<u>Oligonucléotides</u>

Tous les olignucléotides, modifiés ou non, ont été synthétisés par Eurogentec SA, Seraing, Belgique. L'oligoncléotide FAM + DABCYL porte la référence catalogue, OL-0371-0802. Il possède la séquence: GGGTTAGGGTTAGGGTTAGGG correspondant à 3.5 répétitions du motif télomérique humain (brin riche en G). La fluorésceine est attaché à l'extrémité 5', le DABCYL à l'extrémité 3', par les bras chimiques décrit par Eurogentec. La concentration des échantillons est vérifiée par spectrophotométrie, en enregistrant le spectre d'absorbance entre 220 et 700 nm et en utilisant le coefficient d'extinction molaire fourni par le fournisseur.

<u>Tampons</u>

Toutes les expériences ont été réalisées dans un tampon cacodylate de sodium 10 mM pH 7.6 contenant 0.1 M de Chlorure de Lithium (ou de Chlorure de Sodium). L'absence de contamination fluorescente dans le tampon a été préalablement vérifiée. L'oligonucléotide fluorescent est ajouté à la concentration finale de 0.2 μM.

<u>Etude de Fluorescence</u>

Toutes les mesures de fluorescence ont été effectuées sur un appareil Spex Fluorolog DM1 B, en utilisant une largeur de raie d'excitation de 1.8 nm et une largeur de raie d'émission de 4.5 nm. Les échantillons sont placés dans une cuvette en quartz micro de 0.2 x 1 cm. La température de l'échantillon est contrôlée par un bain-marie extérieur. L'oligonucléotide seul a été analysé à 20, 30, 40, 50, 60, 70 et 80°C. Les spectres d'émission sont enregistrés en utilisant une longueur d'onde d'excitation de 470 nm. Les spectres d'excitation sont enregistrés en utilisant soit 515 nm soit 588 nm comme longueur d'onde d'émission. Les spectres sont corrigés de la réponse de l'instrument par des courbes de référence. Une extinction importante (80-90 %) de la fluorescence de la fluoresceine à température ambiante est observée, en accord avec un repli intramoléculaire de l'oligonucléotide à 20°C sous forme d'un G-quadruplex, ce qui induit une juxtaposition de ses extrémités 5' et 3', respectivement liées à la fluoresceine et au DABCYL. Cette juxtaposition entraîne un phénomène déjà décrit d'extinction de fluorescence, utilisé pour les "Molecular Beacons".

Tm en fluorescence

Une solution stock d'oligonucléotide à la concentration en brin de 0.2 µM dans un tampon 0.1 M LiCl 10 mM cacodylate pH 7.6 est préalablement préparée, chauffée brièvement à 90°C et refroidie lentement à 20°C, puis distribuée par aliquots de 600 µl dans les cuves de fluorescence. 3 µl d' eau (pour le contrôle) ou 3 µl du produit à tester (stock à 200 µM, concentration finale 1 µM) sont alors ajoutés et mélangés. Les échantillons sont alors laissés à incuber pendant au moins 1 heure à 20°C avant chaque mesure. L'utilisation de temps d'incubation plus longs (jusqu'à 24 heures) n'a pas d'influence sur le résultat obtenu.

Chaque expérience ne permet que la mesure d'un seul échantillon. Celui ci est d'abord incubé à une température initiale de 20°C, porté à 80°C en 38 minutes, laissé 5 minutes à 80°C, puis refroidi à 20°C en 62 minutes. Durant ce temps, la fluorescence est mesurée simultanément à deux longueurs d'onde d'émission (515 nm et 588 nm) en utilisant 470 nm comme longueur d'onde d'excitation. Une mesure est effectuée toutes les 30 secondes. La température du bain-marie est enregistrée en parallèle, et le profil de fluorescence en fonction de la température est reconstitué à partir de ces valeurs. Les profils de fluorescence sont ensuite normalisés entre 20°C et 80°C, et la température pour laquelle l'intensité d'émission à 515 nm est la moyenne de celles à haute et basse température est appelée Tm. Dans ces conditions, le Tm de l'échantillon de référence sans addition de produit est de 44°C dans un tampon Chlorure de Lithium. Cette température est portée à plus de 55°C dans un tampon Chlorure de Sodium. L'addition d'un composé stabilisant le G-quadruplex induit une augmentation du Tm. Cette augmentation est jugée significative si elle est supérieure à 3°.

L'activité biologique antitélomérase est déterminée par le protocole expérimental suivant :

Préparation de l'extrait enrichi en activité télomérase humaine

La lignée de leucémie HL60 est obtenue auprès de l'ATCC (Americam Type Culture Collection, Rockville USA). Les cellules sont cultivées en suspension dans du milieu RPMI 1640 contenant, L-Glutamine à 2 mM, Pénicilline 200 U/ml, streptomycine 200 µg/ml, gentamycine 50 µg/ml et additionné de 10 % de sérum foetal de veau inactivé par la chaleur.

Une aliquote de $10^5$ cellules est centrifugée à 3000xG et le surnageant écarté. Le culot de cellules est resuspendu par plusieurs pipettages successifs dans 200 µl de tampon de lyse contenant CHAPS 0.5 %, Tris-HCl pH 7,5 10 mM, $MgCl_2$ 1mM, EGTA 1 mM, β-mercaptoéthanol 5 mM, PMSF 0.1 mM et glycérol 10 % et est conservé dans la glace pendant 30 minutes. Le lysat est centrifugé à 16 0000xG pendant 20 minutes à 4°C et 160 µl du surnageant est récupéré. Le dosage des proteines de l'extrait est effectué par la méthode de Bradford. L'extrait est conservé à -80°C.

Dosage de l'activité télomérase

L'inhibition de l'activité télomérase est déterminée par un protocole d'extension de l'oligonucléotide TS ($^{5'}$AATCGTTCGAGCAGAGTT$^{3'}$), en présence d'un extrait cellulaire enrichi en activité télomérase et des composés qui sont ajoutés à différentes concentrations (10, 1, 0.1 et 0,01 µM). La réaction d'extension est suivie d'une amplification PCR des produits d'extension à l'aide des oligonucléotides TS et CXext ($^{5'}$GTGCCCTTACCCTTACCCTTACCCTAA$^{3'}$).

Le milieu réactionnel est préparé selon la composition suivante :

| | |
|---|---|
| Tris HCl pH 8,3 | 20 mM |
| MgCl2 | 1,5 mM |
| Tween 20 | 0,005 % (P/V) |
| EGTA | 1 mM |
| dATP | 50 µM |
| dGTP | 50 µM |
| dCTP | 50 µM |
| dTTP | 50 µM |
| Oligonucléotide TS | 2 µg/ml |
| Oligonucléotide CXext | 2 µg/ml |
| Sérum Albumine bovine | 0,1 mg/ml |
| Taq DNA polymérase | 1 U/ml |
| alpha 32P dCTP (3000 Ci/mmole) | 0.5 µl |
| Extrait télomérase | 200 ng sous un volume de 10 µl |

(suite)

| | |
|---|---|
| Produit à tester ou solvant | sous un volume de 5 µl |
| Eau bi-distillée QS | 50 µl |

Les oligonucléotides sont obtenus auprès d'Eurogentec (Belgique) et sont conservés à -20°C à une concentration stock de 1 mg/ml dans de l'eau distillée.

Les échantillons réactionnels sont assemblés dans des tubes à PCR de 0.2 ml et une goutte d'huile de paraffine est déposée sur chacune des réactions de l'expérience avant la fermeture des tubes.

Les échantillons réactionnels sont ensuite incubés dans un appareil à PCR de type Cetus 4800 selon les conditions de températures suivantes :

15 minutes à 30°C,
1 minute à 90°C,

suivis de 30 cycles de,

30 secondes à 94°C,
30 secondes à 50°C.
et 1 minute 30 secondes à 72°C,

suivis d'un cycle final de 2 minutes à 72°C.

Pour chacun des échantillons, une aliquote de 10 µl est pipettée sous la couche d'huile et mélangée avec 5 µl d'un tampon de dépôt contenant :

| | |
|---|---|
| TBE | 3X |
| glycérol | 32 % (P/V) |
| Bleu de bromophénol | 0.03 % |
| Xylène cyanol | 0.03 % |

Les échantillons sont ensuite analysés par électrophorèse en gel d'acrylamide 12 % dans un tampon TBE 1X pendant 1 heure sous une tension de 200 volts, à l'aide d'un système d'électrophorèse Novex.

Les gels d'acrylamides sont ensuite séchés sur une feuille de papier Whatmann 3 mm à 80°C pendant 1 heure.

L'analyse et la quantification des produits de la réaction sont effectuées à l'aide d'un appareil InstantImager (Pacard).

Pour chaque concentration de composé testée, les résultats sont exprimés en pourcentage d'inhibition de la réaction et calculés à partir du contrôle enzymatique non traité et de l'échantillon sans enzyme (blanc) selon la formule suivante :

(Valeur Composé - valeur blanc/ Valeur contrôle enzymatique -valeur blanc) x 100.

La concentration de composé induisant une inhibition de 50 % de la réaction télomérase (IC50) est déterminée à l'aide d'une représentation graphique semi logarithmique des valeurs d'inhibition obtenues en fonction de chacune des concentrations de composé testée.

On considère qu'un composé est actif en tant qu'agent antitélomérase lorsque la quantité inhibant 50 % de la réaction télomérase est notamment inférieure à 5 µM.

<u>L'activité biologique cytotoxique sur des lignées de tumeur humaines est déterminée selon le protocole expérimental suivant :</u>

Les lignées de cellules humaines A549 sont originaires de l'ATCC (Americam Type Culture Collection, Rockville USA). Les cellules A549 sont cultivées en couche en flacon de culture dans du milieu RPMI 1640, L-Glutamine à 2 mM, Pénicilline 200 U/ml, streptomycine 200 µg/ml et additionné de 10 % de sérum foetal de veau inactivé par la chaleur. Les cellules KB sont cultivées en couche en flacon de culture dans du milieu de Dulbelco's contenant, L-Glutamine à 2 mM, Pénicilline 200 U/ml, streptomycine 200 µg/ml et additionné de 10 % de sérum foetal de veau inactivé par la chaleur.

Les cellules en phase exponentielles de croissances sont trypsinées, lavées dans du PBS 1X et sont ensemencées en microplaques 96 puits (Costar) à raison de $4x10^4$ cellules/ml pour A549 et de $1.5x10^4$ cellules/ml (0.2 ml/puit) puis incubées pendant 96 heures en présence de concentrations variables de produit à étudier (10, 1, 0.1 et 0.01 µM, chaque point en quadruplicata). 16 heures avant la fin de l'incubation, 0.02 % final de rouge neutre est ajouté dans chaque puits. A la fin de l'incubation, les cellules sont lavées par du PBS 1X et lysées par 1 % de lauryl sulfate de sodium. L'incorporation cellulaire du colorant, qui reflète la croissance cellulaire, est évaluée par spectrophotométrie à une longueur d'onde de 540 nm pour chaque échantillon à l'aide d'un appareil de lecture Dynatech MR5000.

Pour chaque concentration de composé testée, les résultats sont exprimés en pourcentage d'inhibition de croissance cellulaire et calculés à partir du contrôle non traité et du milieu de culture sans cellules (blanc) selon la formule suivante :

(Valeur Composé - valeur blanc/Valeur contrôle cellules - valeur

blanc) x 100

La concentration de composé induisant une inhibition de 50 % de la croissance (IC50) est déterminée à l'aide d'une représentation graphique semi logarithmique des valeurs d'inhibition obtenues en fonction de chacune des concentrations de composé testée.

On considère qu'un composé est actif comme agent cytotoxique si la concentration inhibitrice de 50 % de la croissance des cellules tumorales testées est notamment inférieure à 10 µM.

Les exemples suivants et non limitatifs sont donnés pour illustrer l'invention.

**Exemple 1**

Préparation du dichlorure de 2-amino-bis-4,6-[(1'-méthyl-4'-amino-6'-quinaldinio)amino]-triazine

Dans un tricol de 2 $dm^3$, on introduit 200 $cm^3$ d'eau distillée et on charge sous agitation 41,6g (0,16 mol) de chlorhydrate du chlorure de 1-méthyl-4,6-diaminoquinaldinium, qui peut-être obtenu selon J. Chem. Soc., 1953, 50. On obtient une solution limpide jaune foncée où l'on coule 800 $cm^3$ d'éthanol, provoquant un abondant précipité. On porte à 45°C pour dissoudre, puis on ajoute 13,2 g (0,08 mol) de 2-amino-4,6-dichloro-triazine, qui peut être préparée selon J. Amer. Chem. Soc., 1945, 67, 662. Après quelques minutes, un précipité jaune apparaît, et on chauffe à reflux 1 heure. On refroidit et on laisse une nuit en glacière. Le précipité obtenu est filtré et lavé par quatre fois 100 $cm^3$ de d'éthanol aqueux à 80 % puis séché à 45°C. On obtient ainsi 47 g (100 %) de monochlorhydrate du dichlorure de 2-amino-bis-4,6-[(1'-méthyl-4'-amino-6'-quinaldinio)amino]-triazine.

Libération et purification de la forme base

Dans un tricol de 2 $dm^3$, on ajoute 1,2 $dm^3$ d'eau distillée puis 47 g du monochlorhydrate obtenu ci-dessus, on porte à 55°C et on coule 30 $cm^3$ d'ammoniaque concentré (d=0,925), puis on chauffe à 85°C pour favoriser la solubilisation. Un insoluble est filtré à chaud sur 40 g de Supercel et lavé par trois fois 50 $cm^3$ d'eau bouillante. Après concentration du filtrat au demi, et nouvelle filtration sur 10 g de Supercel, on ajoute 1,2 $dm^3$ d'éthanol et on agite 5 minutes, puis laisse au repos une nuit en glacière. Au matin, on filtre, on lave par trois fois 50 $cm^3$ d'éthanol à 66 % et par deux fois 50 $cm^3$ d'éthanol, on sèche sous vide à 45°C, et l'on obtient 34,2 g (79 %) de dichlorure de 2-amino-bis-4,6-[(1'-méthyl-4'-amino-6'-quinaldinio)amino]-triazine brut. Dans un tricol de 2 $dm^3$, on introduit 600 $cm^3$ d'eau distillée et les 34,2 g de base brute, sous agitation on porte à 50°C jusqu'à dissolution presque totale, et on filtre l'insoluble. Le filtrat résultant est chargé dans un tricot de 3 $dm^3$, sous agitation on coule rapidement 1,4 $dm^3$ d'éthanol. Le précipité blanchâtre gélatineux obtenu est filtré, lavé par trois fois 100 $cm^3$ d'éthanol, séché sous vide à 45°C, et l'on obtient 30,7 g (71 %) de dichlorure de 2-amino-bis-4,6-[(1'-méthyl-4'-amino-6'-quinaldinio)amino]-triazine , sous la forme d'un solide blanc dont les caractéristiques sont les suivantes :

- point de fusion = 334°C.
- spectre RMN $^1H$ (300 MHz, $(CD_3)_2SO$ d6, δ en ppm) : 2,72 (s : 6H) ; 4,01 (s : 6H) ; 6,79 (s : 2H) ; 7,09 (mf : 2H) ; 8,11 (d, J = 10 Hz : 2H) ; 8,21 (dd, J = 10 et 2 Hz : 2H) ; de 8,40 à 8,75 (mf étalé : 4H) ; 9,01 (s large : 2H) ; 9,83 (mf : 2H).

**Exemple 2**

Préparation du dichlorure de 2-amino-bis-4.6-[(1'-éthyl-4'-amino-6'-quinaldinio) amino]-triazine

Dans un tricol de 1 $dm^3$, on introduit 75 $cm^3$ d'eau distillée et on charge sous agitation 16,45g (0,06 mol) de chlorhydrate du chlorure de 1-éthyl-4,6-diamino-quinaldinium, qui peut être obtenu selon le brevet U.S. 2 585 905, puis on coule 300 $cm^3$ d'éthanol. On porte à 55°C, puis on ajoute 4,95 g (0,03 mol) 2-amino-4,6-dichloro-triazine et on chauffe à reflux 2 heures et demie. On refroidit et on laisse une nuit en glacière. Le précipité obtenu est filtré, lavé par 100 $cm^3$ d'éthanol à 80 % puis séché à 45°C. On obtient ainsi 16,47 g (91 %) de monochlorhydrate du dichlorure de 2-amino-bis-4,6-[(1 '-éthyl-4'-amino-6'-quinaldinio)amino]-triazine.

Libération et purification de la forme base

Dans un ballon de 250 $cm^3$ contenant 200 $cm^3$ d'eau distillée, sous agitation on charge les 16,47 g de monochlorhydrate obtenu précédemment et on coule 8 $cm^3$ d'ammoniaque concentré (d=0,925), on porte à reflux pour favoriser la solubilisation. On filtre à chaud sur Supercel un léger insoluble qu'on lave par deux fois 10 $cm^3$ d'eau bouillante. Après concentration du filtrat au demi, on ajoute sous agitation 350 $cm^3$ d'éthanol, provoquant un abondant précipité blanc, laissé une nuit en glacière. Le précipité est filtré, lavé par cinq fois 10 $cm^3$ d'éthanol à 80 %, séché sous vide à 55°C, et l'on obtient 12,87 g (76 %) du dichlorure de 2-amino-bis-4,6-[(1'-éthyl-4'-amino-6'-quinaldinio) amino]-triazine, sous forme d'une poudre blanche hygroscopique dont les caractéristiques sont les suivantes :

- point de fusion = 302°C
- spectre RMN $^1H$ (300 MHz, $(CD_3)_2SO$ d6, $\delta$ en ppm) : 1,42 (t, J = 7 Hz : 6H) ; 2,74 (s : 6H); 4,57 (q, J = 7 Hz : 4H) ; 6,80 (s : 2H) ; 7,09 (mf : 2H) ; 8,13 (d, J = 10 Hz : 2H) ; 8,21 (dd, J = 10 et 2 Hz : 2H) ; de 8,40 à 8,75 (mf étalé : 4H) ; 9,01 (s large : 2H) ; 9,83 (mf : 2H).

**Exemple 3**

Préparation du dichlorure de 2-diméthylamino-bis-4,6-[(1'-méthyl-4'-amino-6'-quinaldinio)amino]-triazine

Dans un tricol de 1 $dm^3$, on introduit 60 $cm^3$ d'eau distillée et on charge sous agitation 13,01 g (0,05 mol) du chlorhydrate du chlorure de 1-méthyl-4,6-diamino-quinaldinium, qui peut être obtenu selon J. Chem. Soc., 1953, 50. On obtient une solution jaune où l'on coule 240 $cm^3$ d'éthanol, provoquant un abondant précipité jaune. Après dissolution par chauffage à 50°C, on ajoute 4,83 g (0,025 mol) de 2-diméthylamino-4,6-dichloro-triazine, qui peut être préparée selon J. Amer. Chem. Soc., 1948, 70, 3726. Après quelques minutes, un précipité apparaît et on porte à reflux 1 heure et demie. On refroidit 1 heure dans un bain de glace, puis on filtre le précipité obtenu qui est lavé par quatre fois 30 $cm^3$ d'éthanol et séché. On obtient 12,92 g (86%) de dichlorure de 2-diméthylamino-bis-4,6-[(1'-méthyl-4'-amino-6'-quinaldinio)amino]-triazine, sous forme d'une poudre crème hygroscopique, dont les caractéristiques sont les suivantes :

- point de fusion = 345°C
- spectre RMN $^1H$ (300 MHz, $(CD_3)_2SO$ d6, $\delta$ en ppm) : 2,72 (s : 6H) ; 3,18 (s : 6H) ; 4,00 (s : 6H) ; 6,75 (s : 2H) ; 8,12 (d, J = 9,5 Hz : 2H) ; 8,22 (mt : 2H) ; de 8,40 à 8,65 (mf étalé : 4H) ; 8,79 (s large : 2H) ; 9,83 (mf : 2H).

**Exemple 4**

Préparation du trichlorhydrate de 2-méthylamino-bis-4,6-[(4'-amino-6'-quinaldinyl)amino]-triazine

Dans un tricol de 2 $dm^3$, on introduit 34 $cm^3$ d'eau distillée et 126 $cm^3$ d'acide chlorhydrique normal, et on charge sous agitation 21,82 g (0,1 mol) de 4,6-diaminoquinaldine, qui peut être obtenue selon J. Chem. Soc., 1953, 50. A la solution orangée obtenue, on coule 600 $cm^3$ d'éthanol, on porte à 65°C, puis on ajoute 10,74 g (0,06 mol) de 2-méthylamino-4,6-dichlorotriazine, qui peut être préparée selon Chem. Berichte, 1899, 32, 700, et on coule 40 $cm^3$ d'éthanol. Un précipité jaune apparaît qui s'épaissit rapidement en chauffant à reflux 2 heures. Après refroidissement une nuit en glacière, le précipité obtenu est filtré, lavé par trois fois 120 $cm^3$ d'éthanol à 80 %, séché et on obtient 27,3 g (81 %) de trichlorhydrate de 2-méthylamino-bis-4,6-[(4'-amino-6'-quinaldinyl)amino]-triazine, sous forme d'une poudre blanche hygroscopique dont les caractéristiques sont les suivantes :

- point de fusion = 340°C

- spectre RMN. $^1$H (300 MHz, $(CD_3)_2SO$ d6, δ en ppm) : 2,62 (s large : 6H) ; 2,94 (s large : 3H) ; 6,64 (s large : 2H) ; de 7,60 à 7,80 (mf étalé : 1H) ; 7,89 (d large, J = 9,5 Hz : 2H) ; 8,10 (d large, J = 9,5 Hz : 2H) ; de 8,35 à 8,65 (mf étalé : 4H) ; de 8,70 à 8,95 (mf étalé : 2H) ; de 9,80 à 10,00 (mf étalé : 2H) ; 13,76 (mf : 2H).

**Exemple 5**

Préparation du dichlorure de 2-amino-bis-4.6-[(1'-méthyl-6'-quinoléinio)amino]-triazine

Dans un tricol de 2 $dm^3$, on introduit 225 $cm^3$ d'eau distillée et on charge sous agitation 41,5g (0,18 mol) de chlorhydrate de chlorure de 1-méthyl-6-aminoquirioléinium, qui peut être obtenu selon Zh.Org.Khim. ; 1993, 29(10), 2018.

On obtient une solution jaune où on coule 900 $cm^3$ d'éthanol, provoquant un abondant précipité. Après dissolution par chauffage à 50°C, on ajoute 14,8 g (0,09 mol) de 2-amino-4,6-dichloro-triazine, et on chauffe à reflux 1 heure, un précipité jaune apparaît rapidement. On refroidit une nuit dans la glacière, puis on filtre le précipité obtenu qui est lavé par deux fois 50 $cm^3$ d'éthanol, séché et on obtient 36,6 g (79 %) de monochlorhydrate du dichlorure de 2-amino-bis-4,6-[(1'-méthyl-6'-quinoléinio)amino]-triazine.

Libération et purification de la forme base

Dans un tricol de 1 $dm^3$, on ajoute 200 $cm^3$ d'eau distillée, puis on charge sous agitation les 36,6 g de monochlorhydrate obtenu précédemment, on porte à 80°C et on coule 10 $cm^3$ d'ammoniaque concentré (d=0,925), et on filtre un insoluble sur Supercel. Dans un tricol de 6 $dm^3$, contenant 3 $dm^3$ d'éthanol, on coule sous agitation en 5 minutes le filtrat précédent, un fin précipité jaune vif apparaît, qui est laissé 2 jours en glacière, puis filtré, lavé par deux fois 50 $cm^3$ d'éthanol, séché et on obtient 19,1 g (44%) de dichlorure de 2-amino-bis-4,6-[(1'-méthyl-6'-quinoléinio) amino]-triazine, sous forme d'une poudre jaune hygroscopique dont les caractéristiques sont les suivantes :

- point de fusion = 296°C
- spectre RMN $^1$H (300 MHz, $(CD_3)_2SO$ d6, δ en ppm) : 4,64 (s : 6H) ; 7,11 (mf : 2H) ; 8,10 (dd, J = 8,5 et 6 Hz : 2H) ; 8,46 (d, J = 10 Hz : 2H) ; 8,56 (dd, J = 10 et 2 Hz : 2H) ; 9,17 (d large, J = 8,5 Hz : 2H) ; 9,30 (mt : 4H) ; 10,26 (mf : 2H).

**Exemple 6**

Préparation du trichlorhydrate de 2-méthylamino-bis-4,6-[(4'-méthylamino-6'-quinaldinyl)amino]-triazine

Etape A: Préparation de la 4-méthylamino-6-amino-quinaldine

Dans un tricol de 2 $dm^3$, on introduit 240 $cm^3$ d'acide acétique et on charge sous agitation 57,4g (0,25 mol) de 6-acétamido-4-méthoxyquinaldine préparée selon J. Amer. Chem. Soc., 1948, 70, 4065. On fait barbotter sous agitation de la méthylamine jusqu'à saturation, puis on porte à reflux 2 heures. On refroidit, et fait de nouveau l'opération précédente, on refroidit, et on coule sous agitation la solution obtenue dans un tricol de 2 $dm^3$, contenant 300 $cm^3$ d'eau distillée et 470 $cm^3$ d'acide chlorhydrique normal. On chauffe alors à 100°C pendant 11 heures puis on laisse une nuit en glacière. Le produit cristallisé est filtré, donnant 25 g de chlorhydrate, les liqueurs mères sont concentrées, laissées une nuit en glacière puis filtrées pour donner de nouveau 150 g de chlorhydrate. Les 175 g de chlorhydrate sont repris par 300 $cm^3$ d'eau distillée et dissous par chauffage à 50°C, traités par 1 g de noir et filtrés sur Supercel. On porte le filtrat à 90°C et on alcalinise par addition de 54 $cm^3$ de soude concentrée. Le précipité obtenu par refroidissement la nuit en glacière est lavé par quatre fois 100 $cm^3$ d'eau distillée, séché et on obtient 33 g (70 %) du 4-méthylamino-6-aminoquinaldine

Etape B

Dans un tricol de 2 $dm^3$, on introduit 25 $cm^3$ d'eau distillée et 101 $cm^3$ d'acide chlorhydrique normal, et on charge 18.9 g (0,101 mol) de 4-méthylamino-6-amino quinaldine, la solution orangée obtenue est portée à 75°C, puis on coule en 2 minutes 500 $cm^3$ d'éthanol, et on ajoute d'un coup 8,59 g (0,048 mol) 2-méthylamino-4,6-dichloro-triazine, préparée selon l'exemple 4. Après 5 minutes, un précipité apparaît et on porte à reflux 2 heures, on laisse refroidir sous agitation 3 heures et on abandonne 8 jours en glacière. Le précipité obtenu est filtré, lavé par deux fois 100 $cm^3$ d'éthanol à 80 % et par trois fois 100 $cm^3$ d'éthanol, séché et on obtient 21,7 g (77 %) du trichlorhydrate de 2-méthylamino-bis-4,6-[(4'-méthylamino-6'-quinaldinyl)amino]-triazine, sous forme d'une poudre crème hygroscopique dont

les caractéristiques sont les suivantes :

- point de fusion = 355°C
- spectre RMN. $^{1}H$ (300 MHz, $(CD_3)_2SO$ d6, $\delta$ en ppm) : 2,68 (s : 6H) ; 2,93 (s : 3H) ; 3,04 (mf : 6H) ; 6,59 (s : 2H) ; de 7,40 à 7,70 (mf étalé : 1H) ; 7,88 (d, J = 9 Hz : 2H) ; 8,08 (d large, J = 9 Hz : 2H) ; de 8,50 à 8,95 (mf étalé : 2H) ; de 8,75 à 8,95 (mf : 2H) ; de 9,70 à 10,10 (mf étalé : 2H) ; 13,79 (mf : 2H).

**Exemple 7**

Préparation du chlorhydrate du dichlorure de 2-amino-bis-4,6-[(9'-amino-10'-méthyl-2'-acridinio)amino]-triazine

Etape A : Préparation de la 2-acétamido-9-amino-acridine

Dans un tricol de 250 $cm^3$, on introduit 72 $cm^3$ d'acide acétique et on charge sous agitation 12 g (0,0575 mol) de 2,9-diaminoacridine, préparée selon J. Chem. Soc., 1949, 1148, puis on coule 4,1 $cm^3$ (0,0575 mol) de chlorure d'acétyle. La température monte à 50°C et la solution se prend en masse. On maintient à 60°C pendant 1 heure, on refroidit et on dilue par 200 $cm^3$ d'oxyde de diéthyle. Par filtration, on obtient 15,2 g de chlorhydrate. Dans un ballon de 4 $dm^3$, on introduit 2 $dm^3$ d'eau distillée et on charge sous agitation les 15,2 g de chlorhydrate. On porte à reflux, on filtre et on alcalinise par l'ammoniaque. La base cristallise, est filtrée et séchée. On obtient 11,2 g (78 %) de 2-acétamido-9-amino-acridine.

Etape B : Préparation du sulfate de 2-acétamido-9-amino-10-méthylacridinium

Dans un tricol de 1 $dm^3$, on introduit 300 $cm^3$ de nitrobenzène puis successivement sous agitation 11,2 g (0,0446 mol) de 2-acétamido-9-amino acridine et 11 $cm^3$ (0,116 mol) de sulfate de diméthyle. On porte ensuite à 140°C pendant 20 minutes. Après refroidissement, on filtre le précipité obtenu, qui est lavé par 20 $cm^3$ de nitrobenzène et six fois 20 $cm^3$ d'oxyde de diéthyle, séché à l'air et l'on obtient 14,35 g (85%) de sulfate de 2-acétamido-9-amino-10-méthyl-acridinium.

Etape C : Préparation du chlorhydrate du chlorure de 2-acétamido-9-amino-10-méthyl-acridinium.

Dans un ballon de 2 $dm^3$, on introduit 600 $cm^3$ d'eau distillée et on charge sous agitation 13 g de sulfate de 2-acétamido-9-amino-10-méthyl-acridinium (0,0344 mol), on porte à reflux jusqu'à dissolution presque complète et on filtre l'insoluble. Après refroidissement, on coule 900 $cm^3$ d'une solution aqueuse à 35 % de chlorure de sodium, on laisse précipiter et on filtre. Dans un ballon de 100 $cm^3$, on introduit 20 $cm^3$ d'acide chlorhydrique concentré (d=1,18) et on charge le composé précédent que l'on porte 5 minutes à reflux, on dilue par 67 $cm^3$ d'éthanol et on laisse cristalliser dans la glace. Les cristaux formés sont filtrés, lavés par deux fois 5 $cm^3$ d'éthanol et par trois fois 10 $cm^3$ d'oxyde de diéthyle, séchés et on obtient 5,1 g (50 %) de chlorhydrate du chlorure de 2-acétamido-9-amino-10-méthyl-acridinium.

Etape D

Dans un tricol de 100 $cm^3$, on introduit 5 $cm^3$ d'eau distillée et 37 $cm^3$ d'éthanol et on charge sous agitation 1 g (0,00338 mol) du chlorhydrate du chlorure de 2-acétamido-9-amine-10-méthyl-acridinium, on porte à 80°C et on ajoute 0,28g (0,0017 mol) de 2-amino-4,6-dichloro-triazine. Après 5 minutes, un précipité apparaît et on porte à reflux 2 heures, puis on laisse refroidir, on filtre, on lave par trois fois 3 $cm^3$ d'éthanol et par 5 $cm^3$d'éther, on sèche sous vide et on obtient 0,8 g (73 %) du chlorhydrate de dichlorure de 2-amino-bis-4,6-[(9'-amino-10'-méthyl-2'-acridinio) amino]-triazine, sous forme d'une poudre ocre jaune hygroscopique dont la caractéristique est la suivante :

- point de fusion = 310°C

**Exemple 8**

Préparation du trichlorhydrate de 2-amino-bis-4,6-[(4'-amino-6'-quinaldinyl)amino]-triazine (la base correspondante est aussi appelée SURFENE C)

Dans un tricol de 10 $dm^3$, on introduit 5,25 $dm^3$ d'eau distillée et on charge sous agitation 368,4 g (2,127 mol) de 4,6-diaminoquinaldine, préparée selon l'exemple 4, puis on ajoute 117 g (0,709 mol) 2-amino-4,6-dichloro-triazine. Un précipité jaune se forme immédiatement, on porte à reflux 2 heures. Après refroidissement et acidification à pH=3,4

par addition de 1,3 $dm^3$ d'acide chlorhydrique normal, on porte à reflux 15 minutes, on ajoute 20 g de noir et on maintient le reflux 10 minutes, puis on filtre. Dans un tricol de 10 $dm^3$, on introduit la solution précédente que l'on porte à 80°C, puis on coule en 30 minutes 852 $cm^3$ d'acide chlorhydrique (d=1,19). En fin de coulée, un précipité jaune pâle apparaît, qui, après refroidissement au bain de glace, est filtré, lavé par quatre fois 250 $cm^3$ d'une solution d'acide chlorhydrique, composée de 250 $cm^3$ d'acide chlorhydrique (d=1,19) et de 2 $dm^3$ d'eau distillée, puis séché sous vide à 60°C et on obtient 385,4 g (99 %) trichiorhydrate. Dans un tricol de 4 $dm^3$, on introduit 1,9 $dm^3$ d'éthanol et on charge 385,4 g précédents et on agite 5 heures à température ambiante à l'abri de la lumière, on filtre puis on lave par deux fois 250 $cm^3$ d'éthanol et sèche ; on obtient 346 g (89 %) du trichlorhydrate de 2-amino-bis-4,6-[(4'-amino-6'-qilinaldinyl)amino]-triazine, sous forme d'une poudre crème hygroscopique dont la caractéristique est la suivante :

- point de fusion = 345°C

**Exemple 9**

Trichlorhydrate de 2-amino-bis-4,6-(p-amidinoanilino)-triazine

Ce produit peut être obtenu selon J. Chem. Soc., 1960, 4525 sous forme d'une poudre crème hygroscopique.

**Exemple 10**

Préparation du dichlorure de 2-méthylthio-bis-4,6-[(1'-méthyl-4'-amino-6-quinaldinio)amino]-triazine

Dans un tricol de 1 $dm^3$, on introduit 60 $cm^3$ d'eau distillée et on charge sous agitation 13,01 g (0,05 mol) du chlorhydrate du chlorure de 1-méthyl-4,6-diaminoquinaldinium, qui peut être obtenu selon J. Chem. Soc.,1953, 50. On obtient une solution jaune où l'on coule 240 $cm^3$ d'éthanol, provoquant un abondant précipité jaune. Après dissolution par chauffage à 50°C, on ajoute 4,90 g (0,025 mol) de 2-méthylthio-4,6-dichlorotriazine, qui peut être préparée selon Ang. Chem. Int Ed., 1966, 5, 960. Après quelques minutes, un précipité apparaît et on porte à reflux une heure et demie. On refroidit une nuit à la glacière, puis on filtre le précipité obtenu qui est lavé par quatre fois 30 $cm^3$ d'éthanol et séché. On obtient 10,70 g (75 %) de dichlorure de 2-méthylthio-bis-4,6-[(1'-méthyl-4'-amino-6'-quinaldinio) amino]-triazine, sous forme d'une poudre crème hygroscopique, dont les caractéristiques sont les suivantes :

- point de fusion = 320°C

**Exemple 11**

Préparation du dichlorhydrate dihydrate de 2-chloro-bis-4,6-[(4'-diméthylamino-6'-quinaldinyl)amino]-triazine

Etape A : Préparation de la 4-diméthylamino-6-amino-quinaldine

On opère comme à l'étape A de l'exemple 6, mais à partir de 57,4 g (0,25 mol) de 6-acétamido-4-méthoxy-quinaldine, préparée selon J. Amer. Chem. Soc., 1948, 70, 4065, et de 100 $cm^3$ d'une solution aqueuse à 40 % de diméthylamine dans 250 $cm^3$ d'acide acétique chauffés à 100°C pendant 2 heures dans un autoclave de 1 $dm^3$. On obtient alors, après purification acide-base en opérant comme à l'étape A de l'exemple 6, 38,71g (77 %) de 4-diméthylamino-6-amino-quinaldine.

Etape B

Dans un tricol de 25 $cm^3$, on dissout 402 mg (2 mmoles) de 4-diméthylamino-6-amino-quinaldine dans 7 $cm^3$ d'acide acétique, puis on ajoute en 2 minutes 184 mg (1 mmole) de chlorure de cyanuryle puis on chauffe à 90°C pendant 3 heures. Après refroidissement, les cristaux formés sont essorés, lavés par 2,5 $cm^3$ d'acide acétique et séchés sous vide à 100°C. On obtient ainsi 588 mg (94 %) de dichlorhydrate dihydrate de 2-chloro-bis-4,6-[(4'-diméthylamino-6'-quinaldinyl)amino]-triazine, sous forme de cristaux jaune-pâles dont les caractéristiques sont les suivantes :

- point de fusion = 350°C
- analyse élémentaire : % C = 51,75 (calc = 52,06) ; % H = 5,67 (calc = 5,50) ; % N = 19,98 (calc = 20,23)

**Exemple 12**

Préparation de l'hydrate de 2-méthylthio-bis-4,6-[(4'-diméthylamino-6'-quinaldinyl)amino]-triazine

Dans un tricol de 250 $cm^3$, on introduit 100 $cm^3$ d'éthanol aqueux à 90 % et on charge sous agitation 4,02 g (0,02 mol) de 4-diméthylamino-6-amino-quinaldine préparée à l'étape A de l'exemple 11 et 1,96 g (0,01 mol) de 2-méthylthio-4,6-dichloro-triazine, qui peut être préparée selon Ang. Chem. Int. Ed., 1966, 5, 960. Après quelques minutes, un précipité apparaît et on porte à reflux 1 heure et demie. On refroidit une nuit à la glacière, puis on filtre le précipité obtenu qui est lavé par quatre fois 30 $cm^3$ d'éthanol et séché. On obtient 5,26 g (88 %) de chlorhydrate brut.

Dans un tricol de 250 $cm^3$, on ajoute 75 $cm^3$ d'éthanol aqueux à 90%, puis on charge sous agitation les 5,26 g de chlorhydrate obtenu précédemment, on porte à 80°C et on coule 5 $cm^3$ d'ammoniaque concentré (d = 0,925), et on laisse cristalliser 2 jours à la glacière. On filtre, on lave par deux fois 5 $cm^3$ d'éthanol aqueux à 90 %, on sèche et l'on obtient 3,20 g (59,5 %) d'hydrate de 2-méthylthio-bis-4,6-[(4'-diméthylamino-6'-quinaldinyl) amino]-triazine, sous forme d'un solide jaune pâle dont les caractéristiques sont les suivantes :

- point de fusion = 280-3°C
- analyse élémentaire : % C = 62,06 (calc = 62,48) ; % H = 5,81 (calc = 62,48) ; % N = 23,80 (calc = 23,42).

**Exemple 13**

Préparation du dichlorhydrate de N,N'-(4-amino-6-quinaldinyl)urée (la base correspondante, aussi appelée SURFENE peut être préparée selon Ang. Chem 1939, 891)

Dans un tricol de 500 $cm^3$, on introduit 400 $cm^3$ d'eau distillée et on charge sous agitation 30 g (0,2 mol) de 4,6-diaminoquinaldine, qui peut être préparée selon J. Chem. Soc., 1953, 50, puis on ajoute 8,4g (0,06 mol) d'acétate de sodium trihydrate et on chauffe à 95-96°C. On fait alors passer un courant de phosgène jusqu'à saturation (5 à 10 minutes), puis on maintient à 95-96°C pendant 1 heure. Après refroidissement et acidification par addition de 200 $cm^3$ d'acide chlorhydrique 6 N, le précipité formé est essoré, lavé par 200 $cm^3$ d'acide chlorhydrique N et séché sous vide à 60°C, on obtient ainsi 30 g de chlorhydrate brut, qui est recristallisé dans 300 $cm^3$ d'eau distillée et 30 $cm^3$ d'acide chlorhydrique concentré en présence de noir. Après refoidissement, les cristaux formés sont essorés, lavés à l'acide chlorhydrique N, puis à l'acétone et séché sous vide à 40°C. On obtient ainsi 27 g (60,5 %) du dichlorhydrate de N, N'-(4-amino-6-quinaldinyl)urée, sous forme d'une poudre crème hygroscopique dont la caractéristique est la suivante :

- point de fusion = 329-40°C

**Exemple 14**

Préparation du diodure de $N^1$,$N^5$-bis(7-chloro-1-méthyl-4-quinoléinio) pentane-1,5-diamine

On dissout, par chauffage vers 50°C, 3 g (7 mmoles) de $N^1$,$N^5$-bis(7-chloroquinoléin-4-yl)pentane-1,5-diamine, qui peut être préparée selon J. Med. Chem. 1992, 35, 2129, dans 60 $cm^3$ de butan-2-one. On ajoute 3 g (21 mmoles) d'iodure de méthyle et on porte au reflux pendant 5 heures. Les cristaux formés sont essorés, lavés à la butan-2-one puis à l'oxyde de diéthyle et séchés sous vide. On obtient ainsi 3 g (60 %) de diodure de $N^1$,$N^5$-bis(7-chloro-1-méthyl-4-quinoléinio)pentane-1,5-diamine, sous forme de cristaux beiges dont la caractéristique est la suivante :

- point de fusion = 277-78°C.

**Exemple 15**

Préparation du trichlorhydrate pentahydrate de bis-2,4-[(4'-amino-6'-quinaldinyl)amino]pyrimidine

Dans un tricol de 250 $cm^3$, on porte à reflux 1,73 g (10 mmoles) de 4,6-diamino-1-méthyl-quinoléine, qui peut être obtenue selon J. Chem. Soc., 1953, 50, et 0,74 g de 2,4-dichloropyrimidine dans 75 $cm^3$ d'éthanol et 5 $cm^3$ d'eau pendant 5 heures. Le milieu réactionnel est concentré de moitié, puis laissé à cristalliser à la glacière pendant une nuit. Le précipité formé est essoré, lavé à l'éthanol puis à l'oxyde de diéthyle et séché sous vide. Le précipité obtenu est agité 6 heures dans 10 $cm^3$ d'acide chlorhydrique 0,2 N, puis essoré, lavé à l'eau et séché sous vide à 80°C. On obtient ainsi 0,98 g (46,5 %) de trichlorhydrate pentahydrate de bis-2,4-[(4'-amino-6'-quinaldinyl)amino]pyrimidine, sous forme d'un solide jaune dont les caractéristiques sont les suivantes :

- point de fusion = 310-20°C
- analyse élémentaire : % C = 47,37 (calc = 47,46) ; % H = 5,51 (calc = 5,67) ; % N = 18,41 (calc = 18,45) ; % Cl = 15,84 (calc = 15,76)

**Exemple 16**

Préparation du trichlorhydrate dihydrate de 1,5-(4'-amino-6'-quinaldinyl)biguanide

Dans un tricol de 25 $cm^3$, on ajoute 5 $cm^3$ d'eau, 3 $cm^3$ d'acide chlorhydrique 5N, 1,3 g (7,5 mmoles) de 4,6-diamino-quinaldine, qui peut être obtenue selon J. Chem. Soc., 1953, 50, et 0,34 g (3,75 mmoles) de dicyanamide de sodium et on porte à 50-55°C pendant une nuit. Après refroidissement, le précipité formé est essoré, lavé à l'eau glacée et séché sous vide à 70°C. On obtient ainsi 0,47 g (22,5 %) de trichlorhydrate dihydrate de 1,5-(4'-amino-6'-quinaldinyl)biguanide, sous forme de cristaux jaunes dont les caractéristiques sont les suivantes :

- point de fusion = 262-66°C
- analyse élémentaire : % C = 47,85 (calc = 47,56) ; % H = 5,67 (calc = 5,38) ; % N = 22,96 (calc = 22,69) ; % Cl = 18.79 (calc = 19, 14).

**Exemple 17 : Synthèse en parallèle de dérivés substitués symétriques de 2,4-diamino-6-méthylthio-triazine**

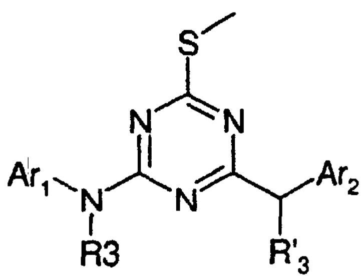

$Ar_1 = Ar_2$

Synthèse en parallèle de N6-[(6-(4-amino-quinaldin-6-yl-amino)-4-méthylthio-triazin-2-yl]-quinaldine-4,6-diamine (exemple 17-1)

Dans un réacteur magnétique chauffant avec condenseur Zymark, de type STEM RS2050 contenant 25 puits en parallèle munis chacun d'un tube en verre de 50 ml, on introduit 50 mg (0,25 mmole) de 2,6-dichloro-6-méthylthio-triazine, qui peut être préparée selon J. Amer. Chem. Soc., 1945, 67, 662. Dans le premier tube (exemple 17-1), on ajoute successivement 5 ml de toluène, 0,5 ml d'une solution N de soude aqueuse et 88 mg (0,5 mmole) de 4,6-diamino-quinaldine. Le milieu réactionnel est chauffé au reflux sous argon pendant 24 heures. Après refroidissement, le contenu du tube est dilué avec 5 ml d'eau et 5 ml de dichlorométhane. La phase organique est décantée, séchée et concentrée sous pression réduite. Le produit brut obtenu est alors purifié par LC/MS en utilisant une colonne de silice C18 Waters Xterra 3.5 µM, de diamètre 3 mm et de longueur 50 mm, en éluant par un gradient linéaire d'élution constitué au temps initial ($t_0$ = 0 mn) par de l'eau contenant 0,05 % d'acide trifluoroacétique et au temps final ($t_1$ = 4 mn) par de l'acétonitrile contenant 0,05 % d'acide trifluoroacétique. On obtient ainsi, après purification, 28 mg de N6-[(6-(4-amino-quinaldin-6-yl-amino)-4-méthylthio-triazin-2-yl]-quinaldine-4,6-diamine, dont les caractéristiques sont les suivantes :

- spectre de masse (DAD-TIC) = 459 ($M^+$)
- temps de rétention = 2,95 mn (dans les conditions décrites ci-dessus pour la purification)

Les exemples 17-1 à 17-3 ont été obtenus en opérant comme ci-dessus dans un réacteur Zymark STEM RS2050. L'exemple 17-1 a été isolé sous forme de base et sous forme de chlorhydrate, les exemples 17-2 et 17-3 ont été isolés uniquement sous forme de chlorhydrate après purification LC/MS par reprise dans une solution 1M d'acide chlorhydrique dans t'oxyde de diéthyle. Les structures, les diverses conditions opératoires utilisées et les caractéristiques des exemples 17-1 à 17-3 sont résumées dans le tableau de résultats.

**Exemple 18 : Synthèse en parallèle de dérivés asymétriquement substitués de N6-(6-amino-4-méthylthio-triazin-2-yl)-quinaldine-4,6-diamine**

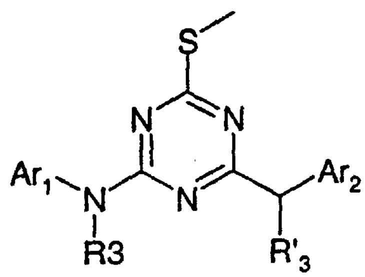

$Ar_1$ différent de $Ar_2$

Préparation de la N6-(6-amino-4-méthylthio-triazin-2-yl)-quinaldine-4,6-diamine

Dans un tricol de 1 litre, à une solution de 5 g (25 mmoles) de 2,6-dichloro-6-méthylthio-triazine, qui peut être préparée selon J. Amer. Chem. Soc., 1945, 67, 662, dans 400 ml de tétrahydrofurane, on ajoute successivement 4,4 g (25 mmoles) de 4,6-diamino-quinaldine et 2,8 g (25 mmoles) de carbonate de sodium. Le mélange réactionnel est chauffé à reflux pendant 16 heures.

Après évaporation du tétrahydrofurane, le résidu est repris par 400 ml d'un mélange d'eau et de dichorométhane (50-50 en volumes). La phase organique est décantée, séchée sur sulfate de sodium et concentrée à sec sous pression réduite. On obtient alors 7,5 g (88 %) de N6-(6-amino-4-méthylthio-triazin-2-yl)-quinaldine-4,6-diamine, sous forme d'un solide jaune pâle dont les caractéristiques sont les suivantes :

- point de fusion = 294°C
- spectre de RMN $^1H$ (300 MHz, $(CD_3)_2SO$ d6, $\delta$ en ppm) : 2,43 (s : 3H) ; 2,52 (s : 3H) ; 6,47 (s : 1H) ; 6,61 (mf : 2H) ; 7,62 (d large, J = 9 Hz : 1H) ; 7,69 (d, J = 9 Hz : 1H) ; 8,32 (mf : 1H) 10,80 (mf : 1H).

Synthèse en parallèle de N6-[(6-(méthyl-quinolin-6-yl-amino)-4-méthylthio-triazin-2-yl]-quinaldine-4,6-diamine (exemple 18-1)

Dans un réacteur magnétique chauffant avec condenseur Zymark, de type STEM RS2050 contenant 25 puits en parallèle munis chacun d'un tube en verre de 50 ml, on introduit 50 mg (0,15 mmole) de N6-(6-amino-4-méthylthio-triazin-2-yl)-quinaldine-4,6-diamine. Dans le premier tube (exemple 18-1), on ajoute successivement 5 ml de dioxane, 16 mg (0.15 mmole) de carbonate de sodium, 23 mg (0,15 mmole) d'iodure de sodium et 49 mg (0,3 mmole) de méthyl-quinolin-6-yl-amine. Le milieu réactionnel est chauffé au reflux sous argon pendant 24 heures. Après refroidissement, le contenu du tube est évaporé sous pression réduite, repris par 5 ml d'eau et 5 ml d'acétate d'éthyle et filtré. La phase organique est décantée, séchée et concentrée sous pression réduite. Le produit brut obtenu est alors purifié par LC/MS en utilisant une colonne de silice C18 Waters Xterra 3.5 µM, de diamètre 3 mm et de longueur 50 mm, en éluant par un gradient linéaire d'élution constitué au temps initial ($t_0$ = 0 mn) par de l'eau contenant 0,05 % d'acide trifluoroacétique et au temps final ($t_1$ = 4 mn) par de l'acétonitrile contenant 0,05 % d'acide trifluoroacétique. On obtient ainsi, après purification, 58 mg de trifluoroacétate de N6-[(6-(méthyl-quinolin-6-yl-amino)-4-méthylthio-triazin-2-yl]-quinaldine-4,6-diamine, dont les caractéristiques sont les suivantes :

- spectre de masse (DAD-TIC)=454($M^+$)
- temps de rétention = 2,51 mn (dans les conditions décrites ci-dessus pour la purification

Les exemples 18-1 à 18-13 ont été obtenus en opérant comme ci-dessus dans un réacteur Zymark STEM RS2050. L'exemple 17-1 peut également être obtenu en opérant comme ci-dessus. Les exemples 18-2 à 18-13 ont été isolés sous forme de base. Les structures, les diverses conditions opératoires utilisées et les caractéristiques des exemples 18-1 à 18-13 sont résumées dans le tableau de résultats.

**<u>Exemple 19</u> : Synthèse en parallèle de dérivés asymétriquement substitués de 2-méthyl-N6-(6-amino-4-diéthylaminotriazin-2-yl)-quinoline-4,6-diamine**

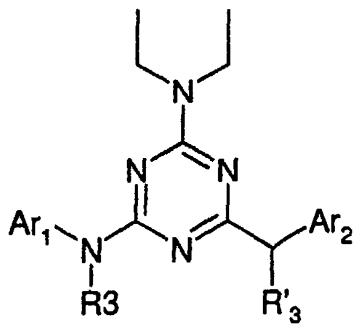

$Ar_1$ différent de $Ar_2$

<u>Préparation de la N6-(6-amino-4-diéthylamino-triazin-2-yl)-quinaldine-4,6-diamine</u>

Dans un tricol de 1 litre, à une solution de 5 g (22,5 mmoles) de 2,6-dichloro-4-diéthylamino-triazine commerciale dans 300 ml de tétrahydrofurane, on ajoute successivement 3,91 g (22,5 mmoles) de 4,6-diamino-quinaldine et 2,4 g (22,5 mmoles) de carbonate de sodium. Le mélange réactionnel est chauffé à reflux pendant 20 heures. Après évaporation du tétrahydrofurane, le résidu est repris par 400 ml d'un mélange d'eau et de dichorométhane (50-50 en volumes). La phase organique est décantée, séchée sur sulfate de sodium et concentrée à sec sous pression réduite. On obtient alors 7,4 g (92 %) de N6-(6-amino-4-diéthylamino-triazin-2-yl)-quinaldine-4,6-diamine, sous forme d'un solide jaune dont les caractéristiques sont les suivantes :

- point de fusion = 120° C
- spectre de RMN $^1H$ (300 MHz, $(CD_3)_2SO$ d6, $\delta$ en ppm) : 1,14 (mt : 6H) ; 2,42 (s : 3H) ; de 3,50 à 3,70 (mt : 4H) ; 6,47 (s et mf : 3H en totalité) ; 7,54 (d large, J = 9 Hz : 1H) ; 7,67(dd, J = 9 et 2 Hz : 1H) ; 8,27 (mf : 1H) ; 10,09 (mf : 1H).

<u>Synthèse en parallèle de N6-[(6-(pyrid-4-yl-amino)-4-diéthylaminotriazin-2-yl]-quinaldine-4,6-diamine</u> (exemple 19-2)

Dans un réacteur magnétique chauffant avec condenseur Zymark, de type STEM RS2050 contenant 25 puits en parallèle munis chacun d'un tube en verre de 50 ml, on introduit 50 mg (0,13 mmole) de N6-(6-amino-4-diéthylamino-triazin-2-yl)-quinaldine-4,6-diamine. Dans le premier tube (exemple 19-2), on ajoute succesivement 5 ml de DMF, 19 mg (0.14 mmole) de carbonate de potassium, 21 mg (0,14 mmole) d'iodure de sodium et 13 mg (0,14 mmole) de pyridin-4-yl-amine. Le milieu réactionnel est chauffé à 120°C sous argon pendant 16 heures. Après refroidissement, le contenu du tube est évaporé sous pression réduite, repris par 5 ml d'eau, filtré et lavé avec de l'oxyde de diéthyle. Le produit brut obtenu est alors purifié par LC/MS en utilisant une colonne de silice C18 Waters Xterra 3.5 µM, de diamètre 3 mm et de longueur 50 mm, en éluant par un gradient linéaire d'élution constitué au temps initial ($t_0$ = 0 mn) par de l'eau contenant 0,05 % d'acide trifluoroacétique et au temps final ($t_l$ = 4 mn) par de l'acétonitrile contenant 0,05 % d'acide trifluoroacétique. On obtient ainsi, après purification, 44 mg de N6-[(6-(pyridin-4-yl-amino)-4-diéthylamino-triazin-2-yl]-quinaldine-4,6-diamine, dont les caractéristiques sont les suivantes :

- spectre de masse (DAD-TIC) = 415 ($M^+$)
- temps de rétention = 0,82 mn (dans les conditions décrites ci-dessus pour la purification

Les exemptes 19-1 à 19-2 ont été obtenus en opérant comme ci-dessus dans un réacteur Zymark STEM RS2050. L'exemple 19-1 a été isolé sous forme sous forme de chlorhydrate après purification LC/MS par reprise dans une solution 1M d'acide chlorhydrique dans l'oxyde de diéthyle. Les structures, les diverses conditions opératoires utilisées et les caractéristiques des exemples 19-1 et 19-2 sont résumées dans le tableau de résultats.

**Exemple 20**

Les activités G-quartet, antitélomérase et cytotoxique des différents composés exemplifiés sont déterminées selon les protocoles opératoires décrits ci-avant.

| EXEMPLE | FLUORESCENCE Tm (°C) | Télomérase IC50(μM) | Cytotox. A549 IC50 (μM) |
|---|---|---|---|
| 1 | - | 0,25 | 0,59/1,9* |
| 2 | 48° | 0,056 | 4,7 |
| 3 | 52° | 0,22 | - |
| 4 | 48° | 0,51 | 3,1 |
| 5 | 57° | 0,13 | 0,56/1,8* |
| 6 | 44° | 0,3 | 1,9 |
| 7 | 55° | 0,89 | 4,9 |
| 8 | - | 0,051 | 9,1 |
| 9 | - | 0,74 | 0,53 |
| 10 | - | 0,24 | 3,6 |
| 11 | 57° | 3 | 5,14 |
| 12 | 70° | 0,041 | 0,44/1,1* |
| 13 | - | 0,72 | - |
| 14 | - | 1,4/4,9* | 6,5 |
| 15 | 53° | 0,49 | 8,7 |
| 16 | 52° | 2/4,5* | 5,8 |
| 17-1 | 57 | 0,049 | 1,6 |
| 17-2 | - | 0,95 | 6,1 |
| 17-3 | 6 | 3,9 | 3,2 |
| 18-1 | 47 | 3,5 | - |
| 18-2 | 49 | 2,3 | - |
| 18-3 | 57 | 0,34 | 10 |
| 18-5 | 47 | 3,4 | - |
| 18-6 | 50 | 2,6 | 10 |
| 18-7 | 51 | 3,3 | 8 |
| 18-8 | 49 | 3,4 | - |
| 18-9 | 51 | 3,4 | 9,5 |
| 18-10 | 49 | 3,3 | 10 |
| 18-11 | 48 | 3,3 | 1,3 |
| 18-12 | - | 2,8 | - |
| 18-13 | - | 3,4 | - |
| 19-1 | 58 | 1,0 | 1,5 |
| 19-2 | 53 | 2,5 | - |

* : résultats de deux expériences indépendantes

| Exemple | Structures | | | | | Conditions réactionnelles | | | Caractéristiques | |
|---|---|---|---|---|---|---|---|---|---|---|
| | A | $Ar_1$ | $R_3$ | $Ar_2$ | $R'_3$ | Solvant | Chauffage | Nbre de mmoles d'amine | Masse $M^+$ | Temps de rétention |
| 17-1 | SMe | 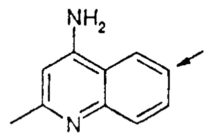 | H | 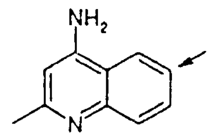 | H | toluène | 24 h./110° | 0,5 | 469 | 2,95 mn |
| 17-2 | SMe | 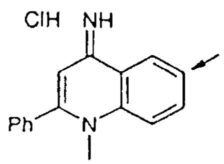 | H | 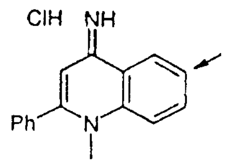 | H | toluène | 48 h./110° | 0,75 | 621 | 2,21 mn |
| 17-3 | SMe | 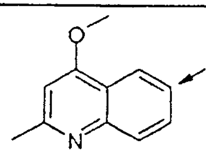 | H | 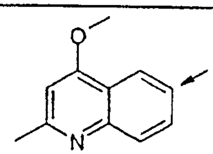 | H | toluène | 24 h./100° | 0,5 | 499 | 3,02 mn |
| 18-1 | SMe | 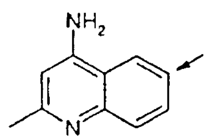 | H | 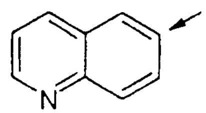 | Me | dioxane | 24 h./100° | 0,3 | 454 | 2,51 mn |

| Exemple | Structures | | | | | Conditions réactionnelles | | | Caractéristiques | |
|---|---|---|---|---|---|---|---|---|---|---|
| | A | $Ar_1$ | $R_3$ | $Ar_2$ | $R'_3$ | Solvant | Chauffage | Nbre de mmoles d'amine | Masse $M^+$ | Temps de rétention |
| 18-2 | SMe | 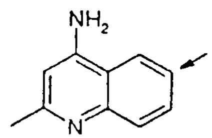 | H | 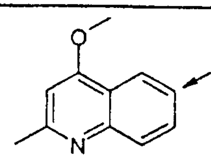 | H | dioxane | 18 h../100° | 0,3 | 484 | 2,91 mn |
| 18-3 | SMe | 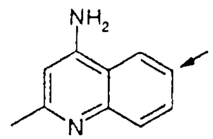 | H | 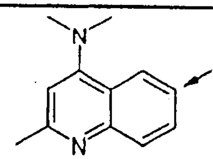 | H | dioxane | 24 h./100° | 0,3 | 497 | 3,07 mn |
| 18-4 | SMe | 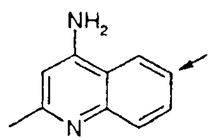 | H | 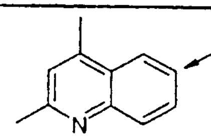 | H | dioxane | 24 h./100° | 0,3 | 468 | 3,02 mn |
| 18-5 | SMe | 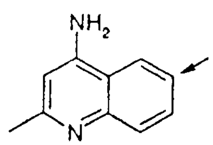 | H | 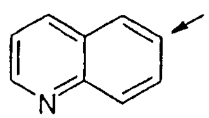 | H | dioxane | 48 h./100° | 0,3 | 440 | 3,20 mn |

| Exemple | Structures | | | | | Conditions réactionnelles | | | Caractéristiques | |
|---|---|---|---|---|---|---|---|---|---|---|
| | A | $Ar_1$ | $R_3$ | $Ar_2$ | $R'_3$ | Solvant | Chauffage | Nbre de mmoles d'amine | Masse $M^+$ | Temps de rétention |
| 18-6 | SMe | 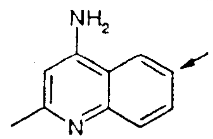 | H | 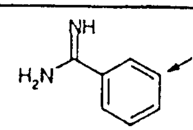 | H | dioxane | 48 h./100° | 0,3 | 431 | 2,85 mn |
| 18-7 | SMe | 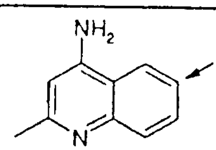 | H | 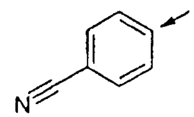 | H | DMF | 96 h./100° | 0,6 | 414 | 3,60 mn |
| 18-8 | SMe | 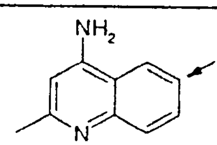 | H | 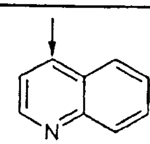 | H | dioxane | 48 h./100° | 0,3 | 440 | 2,94 mn |
| 18-9 | SMe | 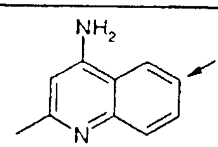 | H | 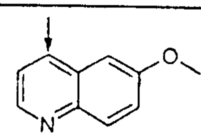 | H | dioxane | 72 h./100° | 0,45 | 470 | 3,05 mn |
| 18-10 | SMe | 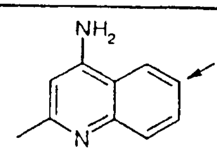 | H | 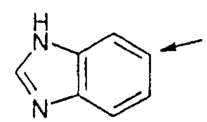 | H | dioxane | 72 h./100° | 0,45 | 429 | 2,84 mn |

| Exemple | Structures | | | | | Conditions réactionnelles | | | Caractéristiques | |
|---|---|---|---|---|---|---|---|---|---|---|
| | A | $Ar_1$ | $R_3$ | $Ar_2$ | $R'_3$ | Solvant | Chauffage | Nbre de mmoles d'amine | Masse $M^+$ | Temps de rétention |
| 18-11 | SMe | 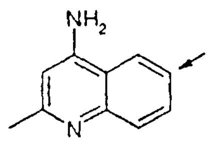 | H | 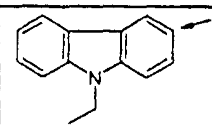 | H | dioxane | 24 h./100° | 0,15 | 506 | 4,06 mn |
| 18-12 | SMe | 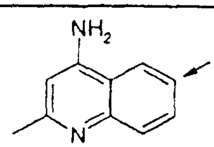 | H | 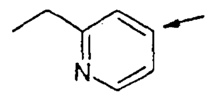 | H | dioxane | 48 h./100° | 0,3 | 418 | 2,85 mn |
| 18-13 | SMe | 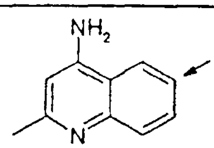 | H | 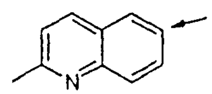 | H | dioxane | 72 h./100° | 0,45 | 454 | 2,94 mn |
| 19-1 | $N(Et)_2$ | 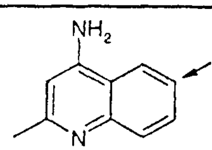 | H | 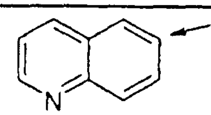 | H | DMF | 16 h./120° | 0,21 | 465 | 1,03 mn |
| 19-2 | $N(Et)_2$ | 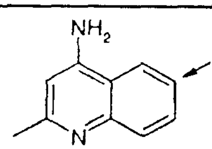 | H | 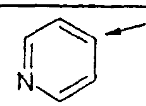 | H | DMF | 16 h./120° | 0,14 | 415 | 0,82 mn |

## Revendications

**1.** Composés fixant la structure G-quadruplex des télomères **caractérisés en ce qu'**ils répondent à la formule générale suivante :

cycle aromatique azoté - $NR_3$ - répartiteur- $NR'_3$ - cycle aromatique

dans laquelle

- le cycle aromatique azoté, représente :
  - ◊ une quinoléine éventuellement substituée par au moins
    - un groupe N(Ra)(Rb) dans lequel Ra et Rb, identiques ou différents représentent l'hydrogène ou un radical alkyle en C1-C4 ou
    - un groupe alkoxy à chaîne courte en C1-C4 ou
  - ◊ une quinoléine possédant un atome d'azote sous forme quaternaire ou
  - ◊ une benzamidine ou
  - ◊ une pyridine
- le cycle aromatique représente
  - ◊ une quinoléine éventuellement substituée par au moins
    - un groupe N(Ra)(Rb) dans lequel Ra et Rb, identiques ou différents représentent l'hydrogène ou un radical alkyle en C1-C4 ou
    - un groupe alkoxy à chaîne courte en C1-C4 ou
  - ◊ une quinoléine possédant un atome d'azote sous forme quaternaire ou
  - ◊ une benzamidine ou
  - ◊ une pyridine ou
  - ◊ un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4 ou
  - ◊ un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4
- R3 et R'3, identiques ou différents, représentent indépendamment l'un de l'autre l'hydrogène ou un radical alkyle en C1-C4
- le répartiteur représente :
  - ◊ un groupe triazine éventuellement substitué par un radical alkyle ayant 1 à 4 atomes de carbone, un radical thio, oxy ou amino eux même éventuellement substitués par un ou plusieurs chaines alkyle à chaine courte contenant 1 à 4 atomes de carbone ou un atome d'halogène ou
  - ◊ un groupe carbonyle ou
  - ◊ un groupe C(=NH)-NH-C(=NH) ou
  - ◊ un groupe alkylediyle contenant 3 à 7 atomes de carbone ou
  - ◊ un groupe diazine éventuellement substitué par les mêmes groupes que la triazine

ou un de ses sels.

**2.** Composés selon la revendication 1 **caractérisés en ce que** le répartiteur est choisi parmi les groupes triazine ou diazine.

**3.** Composés selon la revendication 2 **caractérisés en ce que** les groupes diazines sont des pyrimidines.

**4.** Composés selon la revendication 1 **caractérisés en ce qu'**ils répondent à la formule (I) ci-dessous :

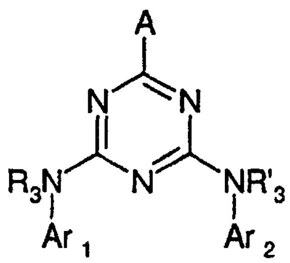

dans laquelle :

- A représente
  - un groupe amino de formule NR1R2 dans lequel R1 et R2 identiques ou différents représentent l'hydrogène ou un groupe alkyle droit ou ramifié contenant 1 à 4 atomes de carbone ou
  - un groupe OR1 ou SR1 dans lequel R1 a la même signification que précédemment ou
  - un groupe alkyle contenant 1 à 4 atomes de carbone ou ou un groupe trifluorométhyle ou
  - un atome d'hydrogène ou
  - un atome d'halogène choisi parmi le fluor, le chlore, le brome ou l'iode
- R3 et R'3, identiques ou différents, représentent indépendamment l'un de l'autre l'hydrogène ou un groupe alkyle en C1-C4
- $Ar_1$ et $Ar_2$ identiques ou différents représentent

  1. quand $Ar_1$ et $Ar_2$ sont identiques :
     - un motif quinoléine éventuellement substitué par au moins
       - un groupe N(Ra)(Rb) dans lequel Ra et Rb identiques ou différents représentent l'hydrogène ou un radical alkyle en C1-C4 ou
       - un groupe alkoxy à chaîne courte contenant 1 à 4 atome de carbone ou
     - une quinoléine possédant un atome d'azote sous forme quaternaire ou
     - une benzamidine ou
     - une pyridine attachée en position -4 ou fusionnée avec un groupe aryle ou hétéroaryle éventuellement substituée par un groupe alkyle en C1-C4

  2. quand $Ar_1$ et $Ar_2$ sont différents
     - $Ar_1$ et $Ar_2$ représentent tous les deux l'une des possibilités évoquées ci-dessus pour $Ar_1$ et $Ar_2$ ou
     - $Ar_1$ représente l'une des possibilités ci-dessus et $Ar_2$ représente

- * un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4
- * un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4

ou un de ses sels.

5. Composés selon la revendication 3 **caractérisés en ce que** $Ar_1$ et $Ar_2$ représentent un groupe choisi parmi les groupes suivants : 4-amino- ou 4-méthylamino- ou 4-diméthylamino- quinolyl ou quinolinium dont le noyau quinolinium est éventuellement substitué par un goupe méthyle.

6. Composés selon la revendication 1 **caractérisé en ce que** le groupe A représente le radical thiométhyl, amino, alkylamino ou dialkylamino radicaux dans lesquels les groupes alkyle possèdent 1 à 4 atomes de carbone.

7. Composés selon la revendication 2 **caractérisés en ce que** A représente un groupe méthylthio.

8. Composés de la revendication 1 **caractérisés en ce qu'**ils ont une activité inhibitrice des télomérases.

9. Composés selon l'une quelconque des revendications précédentes **caractérisés en ce qu'**ils ont une activité anticancéreuse.

10. Composés nouveaux répondant à la formule (I) suivante :

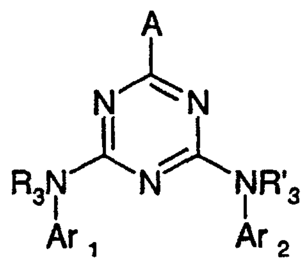

dans laquelle :

- A représente
  - un groupe amino de formule NR1R2 dans lequel R1 et R2 identiques ou différents représentent un groupe alkyle droit ou ramifié contenant 1 à 4 atomes de carbone ou
  - un groupe OR1 ou SR1 dans lequel R1 représente l'hydrogène ou a la même signification que précédemment ou
  - un groupe alkyle contenant 1 à 4 atomes de carbone ou un groupe trifluorométhyle ou
  - un atome d'hydrogène ou
  - un atome d'halogène choisi parmi le fluor, le chlore, le brome ou l'iode
- R3 et R'3, identiques ou différents, représentent indépendamment l'un de l'autre un atome d'hydrogène ou un groupe alkyle en C1-C4
- $Ar_1$ et $Ar_2$ identiques ou différents représentent

1. quand $Ar_1$ et $Ar_2$ sont identiques :

- un motif quinoléine éventuellement substitué par au moins
  - un groupe N(Ra)(Rb) dans lequel Ra et Rb identiques ou différents représentent l'hydrogène ou un radical alkyle en C1-C4 ou
  - un groupe alkoxy à chaîne courte contenant 1 à 4 atome de carbone ou
- une quinoléine possédant un atome d'azote sous forme quaternaire ou
- une benzamidine sauf dans le cas où A représente la diéthylamine, l'hydrogène ou un groupe amine
- une pyridine attachée en position -4 ou fusionnée avec un groupe aryle ou hétéroaryle éventuellement substituée par un groupe alkyle en C1-C4

2. quand $Ar_1$ et $Ar_2$ sont différents

- $Ar_1$ et $Ar_2$ représentent tous les deux l'une des possibilités évoquées ci-dessus pour $Ar_1$ et $Ar_2$ ou
- $Ar_1$ représente l'une des possibilités ci-dessus et $Ar_2$ représente
  * un noyau phényle éventuellement substitué par un groupement halogène, alkoxy en C1-C4, cyano, carbonylamino éventuellement substitué par un ou plusieurs groupes alkyle en C1-C4, guanyl, alkylthio en C1-C4, amino, alkylamino en C1-C4, dialkylamino en C1-C4 pour chaque groupe alkyle, nitro, alkylèneamino en C1-C4 ou alkénylèneamino en C2-C4
  * un noyau hétérocyclique mono ou bi ou tricyclique comportant 0 à 2 hétéroatome par cycle à la condition qu'au moins un hétéroatome soit présent dans au moins un cycle éventuellement substitué par un ou plusieurs groupes alkyle en C1-C4 ou par des groupes alkylène en C1-C4 ou alkénylène en C2-C4

ou un de ses sels à l'exclusion du dichlorhydrate de la 2-amino-bis-4,6-[(4'-amino-6'-quinaldinyl)amino]-triazine et de la 2-amino-bis-4,6-(p-amidino-anilino)-triazine.

**11.** Composés selon la revendication 10 caractérisés en ce quand $Ar_1$ et $Ar_2$ sont identiques, $Ar_1$ et $Ar_2$ représentent un groupe choisi parmi les groupes 4-amino- ou 4-méthylamino- ou 4-diméthylamino- quinolyl ou quinolinium dont le noyau quinolinium est éventuellement substitué par un goupe méthyle.

**12.** Composés selon la revendication 10 **caractérisés en ce que** R1 et R2 représentent l'hydrogène.

**13.** Composés selon la revendication 10 **caractérisés en ce que** A représente un groupe méthylthio.

**14.** Composés selon la revendication 10 caractérisés en ce quand $Ar_1$ et $Ar_2$ sont différents

1. $Ar_1$ représente :

- un motif quinoléine substitué par au moins
  - un groupe N(Ra)(Rb) dans lequel Ra et Rb identiques ou différents représentent l'hydrogène ou un radical alkyle en C1-C4 ou
  - un groupe alkoxy à chaîne courte contenant 1 à 4 atome de carbone ou
- une quinoléine possédant un atome d'azote sous forme quaternaire ou
- une benzamidine sauf dans le cas où A représente la diéthylamine, l'hydrogène ou un groupe amine ou
- une pyridine attachée en position -4 ou fusionnée avec un groupe aryle ou hétéroaryle

2. $Ar_2$ représente

* un noyau tel que défini ci-dessus mais différent ou

* un noyau phényle éventuellement substitué par un groupement halogène, méthoxy, cyano, carbonylamino, guanyl, méthylthio, amino, méthylamino, diméthylamino, morpholine, alkylèneamino en C1-C4 ou alkénylèneamino en C2-C4

* un noyau quinoline, benzimidazole, indole, benzothiophène, benzofurane, benzothiazole, benzoxazole, carbazole, quinazoline, quinoxaline éventuellement substitué par un ou plusieurs groupes alkyle en C1-C4 ou par des groupes alkylène en C1-C4 ou alkénylène en C2-C4

ou un de ses sels à l'exclusion du dichlorhydrate de la 2-amino-bis-4,6-[(4'-amino-6'-quinaldinyl)amino]-triazine et de la 2-amino-bis-4,6-(p-amidino-anilino)-triazine.

**15.** Composés selon la revendication 10 choisis parmi :

- le dichlorure de 2-amino-bis-4,6-[(1'-méthyl-4'-amino-6'-quinaldinio) amino]-triazine
- le dichlorure de 2-amino-bis-4,6-[(1'-éthyl-4'-amino-6'-quinaldinio) amino]-triazine
- le dichlorure de 2-diméthylamino-bis-4,6-[(1'-méthyl-4'-amino-6'-quinaldinio)amino]-triazine
- le trichlorhydrate de 2-méthylamino-bis-4,6-[(4'-amino-6'-quinaldinyl) amino]-triazine
- le dichlorure de 2-amino-bis-4,6-[(1'-méthyl-6'-quinoléinio)amino]-triazine
- le trichlorhydrate du dichlorure de 2-méthylamino-bis-4,6-[(4'-méthylamino-6'-quinaldinyl)amino]-triazine
- le chlorhydrate du dichlorure de 2-amino-bis-4,6-[(9'-amino-10'-méthyl-2'-acridinio)amino]-triazine
- le dichlorure de 2-méthylthio-bis-4,6-[(1'-méthyl-4'-amino-6'-quinaldinio)amino]-triazine
- le dichlorhydrate dihydrate de 2-chloro-bis-4,6-[(4'-diméthylamino-6'-quinaldinyl)amino]-triazine
- l'hydrate de 2-méthylthio-bis-4,6-[(4'-diméthylamino-6'-quinaldinyl) amino]-triazine
- le dichlorhydrate de N,N'-(4-amino-6-quinaldinyl)urée
- le diodure de $N^1$,$N^5$-bis(7-chloro-1-méthyl-4-quinoléinio)pentane-1,5-diamine
- le trichlorhydrate pentahydrate de bis-2,4-[(4'-amino-6'-quinaldinyl) amino]pyrimidine
- le trichlorhydrate dihydrate de 1,5-(4'-amino-6'-quinaldinyl)biguanide
- le 6-[4-(4-amino-2-méthyl-quinolin-6-ylamlno)-6-méthylsulfanyl-[1,3,5]triazin-2-ylamino]-2-méthyl-quinolin-4-ol
- la N6-[4-(4-diméthylamino-2-méthyl-quinolin-6-ylamino)-6-méthyl-sulfanyl-[1,3,5]triazin-2-yl]-2-méthyl-quinolin-4,6-diamine
- la N6-[4-(4-amino-2-méthyl-quinolin-6-ylamino)-6-méthylsulfanyl-[1,3,5]triazin-2-yl]-2-méthyl-quinolin-4,6-diamine
- la N6-[4-(4-méthoxy-2-méthyl-quinolin-6-ylamino)-6-méthylsulfanyl-[1,3,5]triazin-2-yl]-4-méthoxy-2-méthyl-quinolin-6-amine
- la N6-[(6-(4-amino-quinaldin-6-yl-amino)-4-méthylthio-triazin-2-yl]-quinaldine-4,6-diamine
- la N6-[(6-(4-diméthylamino-quinaldin-6-yl-amino)-4-méthylthiotriazin-2-yl]-quinaldiné-4,6-diamine
- la N6-[(6-(quinolyl-6-yl-amino)-4-diethylamino-triazin-2-yl]-quinaldine-4,6-diamine

**16.** Composés selon la revendication 15 choisis parmi :

- l'hydrate de 2-méthylthio-bis-4,6-[(4'-diméthylamino-6'-quinaldinyl) amino]-triazine

- le dichlorhydrate dihydrate de 2-chloro-bis-4,6-[(4'-diméthylamino-6'-quinaldinyl)amino]-triazine

- le 6-[4-(4-amino-2-méthyl-qulnolin-6-ylamino)-6-méthylsulfanyl-[1,3,5]triazin-2-ylamino]-2-méthyl-quinolin-4-ol

- la N6-[4-(4-diméthylamino-2-méthyl-quinolin-6-ylamlno)-6-méthyl-sulfanyl-[1,3,5]triazin-2-yl]-2-méthyl-quinolin-4,6-diamine

- la N6-[4-(4-amino-2-méthyl-quinolin-6-ylamino)-6-méthylsulfanyl[1,3,5]triazin-2-yl]-2-méthyl-quinolin-4,6-diamine

- la N6-[4-(4-méthoxy-2-méthyl-quinolin-6-ylamino)-6-méthylsulfanyl-[1,3,5]triazin-2-yl]-4-méthoxy-2-méthyl-quinolin-6-amine

- la N6-[(6-(4-amino-quinaldin-6-yl-amino)-4-méthylthio-triazin-2-yl]-quinaldine-4,6-diamine

- la N6-[(6-(4-diméthylamino-quinaldin-6-yl-amino)-4-méthylthiotriazin-2-yl]-quinaldine-4,6-diamine
- la N6-[(6-(quinolyl-6-yl-amino)-4-diethylamino-triazin-2-yl]-quinaldine-4,6-diamine

**17.** Utilisation des composés de la revendication 10 comme produit pharmaceutique à usage humain.

**18.** Associations thérapeutiques constituées d'un composé selon la revendication 1 et d'un autre composé anticancéreux.

**19.** Associations selon la revendication 18 **caractérisées en ce que** le composé anticancéreux est choisi parmi les agents alkylants, les dérivés du platine, les agents antibiotiques, les agents antimicrotubules, les anthracyclines, les topoisomérases des groupes I et II, les fluoropyrimidines, les analogues de cytidine, les analogues d'adénosine, les enzymes et composés divers tels que la L-asparaginase, l'hydroxyurée, l'acide trans-rétinoique, la suramine, l'irinotecan, le topotecan, la dexrazoxane, l'amifostine, l'herceptin ainsi que les hormones oetrogéniques, androgéniques, les agents antivasculaires.

**20.** Association thérapeutique constituée d'un composé selon la revendication 1 et de radiations.

**21.** Associations selon l'une quelconque des revendications 18 à 20 **caractérisées en ce que** chacun des composés ou des traitements est administré simultanément, séparément ou séquentiellement.

## Patentansprüche

**1.** Verbindungen zur Fixierung der Struktur. G-Quadruplex von Telomeren, **dadurch gekennzeichnet, daß** sie der folgenden allgemeinen Formel entsprechen:

aromatischer Stickstoffring - $NR_3$- Verteiler - $NR'_3$ - aromatischer Ring

worin

- der aromatische Stickstoffring darstellt :
  - ◊ ein Chinolin, gegebenenfalls substituiert durch mindestens
    - eine Gruppe N(Ra)(Rb), worin Ra und Rb, gleich oder verschieden, Wasserstoff oder einen Rest Alkyl mit 1 bis 4 Kohlenstoffatomen darstellen, oder
    - eine Gruppe Alkoxy mit kurzer Kette von 1 bis 4 Kohlenstoffatomen, oder
  - ◊ ein Chinolin mit einem Stickstoffatom in quaternärer Form oder
  - ◊ ein Benzamidin oder
  - ◊ ein Pyridin
- der aromatische Ring darstellt :
  - ◊ ein Chinolin, gegebenenfalls substituiert durch mindestens
    - eine Gruppe N(Ra)(Rb), worin Ra und Rb, gleich oder verschieden, Wasserstoff oder einen Rest Alkyl mit 1 bis 4 Kohlenstoffatomen darstellen, oder
    - eine Gruppe Alkoxy mit kurzer Kette von 1 bis 4 Kohlenstoffatomen, oder
  - ◊ ein Chinolin mit einem Stickstoffatom in quaternärer Form oder
  - ◊ ein Benzamidin oder

◊ ein Pyridin oder
◊ einen Phenylkern, gegebenenfalls substituiert durch eine Gruppe Halogen, Alkoxy mit 1 bis 4 Kohlenstoffatomen, Cyano, Carbonylamino, gegebenenfalls substituiert durch eine oder mehrere Gruppen Alkyl mit 1 bis 4 Kohlenstoffatomen, Guanyl, Alkylthio mit 1 bis 4 Kohlenstoffatlomen, Amino, Alkylamino mit 1 bis 4 Kohlenstoffatomen, Dialkylamino mit 1 bis 4 Kohlenstoffatomen für jede Gruppe Alkyl, Nitro, Alkylenamino mit 1 bis 4 Kohlenstoffatomen oder Alkenylenamino mit 2 bis 4 Kohlenstoffatomen oder
◊ einen mono- oder bi- oder tricyclischen heterocyclischen Kern mit 0 bis 2 Heteroatomen pro Ring, unter der Bedingung, daß mindestens ein Heteroatom in mindestens einem Ring anwesend ist, gegebenenfalls substituiert durch eine oder mehrere Gruppen Alkyl mit 1 bis 4 Kohlenstoffatomen oder durch Gruppen Alkylen mit 1 bis 4 Kohlenstoffatomen oder Gruppen Alkenylen mit 2 bis 4 Kohlenstoffatomen,

- $R_3$ und $R'_3$, gleich oder verschieden, unabhängig voneinander Wasserstoff oder einen Rest Alkyl mit 1 bis 4 Kohlenstoffatomen bedeutet,

- der Verteiler darstellt :

◊ eine Gruppe Triazin, gegebenenfalls substituiert durch einen Rest Alkyl mit 1 bis 4 Kohlenstoffatomen, einen Rest Thio, Oxy oder Amino, die gegebenenfalls selbst substituiert sind durch eine oder mehrere Ketten Alkyl mit kurzer Kette von 1 bis 4 Kohlenstoffatomen oder ein Halogenatom, oder
◊ eine Gruppe Carbonyl oder
◊ eine Gruppe C(=NH)-NH-C(=NH) oder
◊ eine Gruppe Alkyldiyl mit 3 bis 7 Kohlenstoffatomen oder
◊ eine Gruppe Diazin, gegebenenfalls substituiert durch die gleichen Gruppen wie beim Triazin,

oder eines ihrer Salze.

**2.** Verbindungen nach Anspruch 1, **dadurch gekennzeichnet, daß** der Verteiler unter den Gruppen Triazin oder Diazin ausgewählt wird.

**3.** Verbindungen nach Anspruch 2, **dadurch gekennzeichnet, daß** die Gruppen Diazin Pyrimidine sind.

**4.** Verbindungen nach Anspruch 1, **dadurch gekennzeichnet, daß** sie der nachstehenden Formel (I) entsprechen :

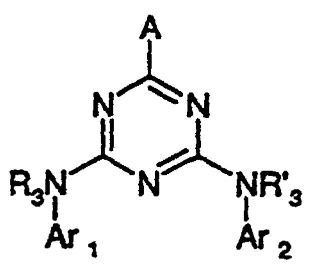

(I)

in der

- A darstellt :

  - eine Gruppe Amino der Formel $NR_1R_2$, worin $R_1$ und $R_2$, gleich oder verschieden, Wasserstoff oder eine gerade oder verzweigte Gruppe Alkyl mit 1 bis 4 Kohlenstoffatomen darstellen, oder
  - eine Gruppe $OR_1$ oder $SR_1$, worin $R_1$ die gleiche Bedeutung wie vorstehend besitzt, oder
  - eine Gruppe Alkyl mit 1 bis 4 Kohlenstoffatomen oder eine Gruppe Trifluormethyl, oder
  - ein Wasserstoffatom oder
  - ein Halogenatom, ausgewählt unter Fluor, Chlor, Brom oder Iod,

- $R_3$ und $R'_3$, gleich oder verschieden, unabhängig voneinander Wasserstoff oder eine Gruppe Alkyl mit 1 bis 4 Kohlenstoffatomen darstellen,
- $Ar_1$ und $Ar_2$, gleich oder verschieden, darstellen :

1. wenn $Ar_1$ und $Ar_2$ gleich sind :

- eine Struktureinheit Chinolin, gegebenenfalls substituiert durch mindestens

  - eine Gruppe N(Ra)(Rb), worin Ra und Rb, gleich oder verschieden, Wasserstoff oder einen Rest Alkyl mit 1 bis 4 Kohlenstoffatomen darstellen, oder
  - eine Gruppe Alkoxy mit kurzer Kette von 1 bis 4 Kohlenstoffatomen, oder

- ein Chinolin mit einem Stickstoffatom in quaternärer Form oder
- ein Benzamidin oder
- ein Pyridin, gebunden in Position -4 oder verbunden mit einer Gruppe Aryl oder Heteroaryl, gegebenenfalls substituiert durch eine Gruppe Alkyl mit 1 bis 4 Kohlenstoffatomen,

2. wenn $Ar_1$ und $Ar_2$ verschieden sind :

- $Ar_1$ und $Ar_2$ beide eine der oben bei $Ar_1$ und $Ar_2$ erwähnten Möglichkeiten darstellen, oder

- $Ar_1$ eine der obigen Möglichkeiten bedeutet und $Ar_2$ darstellt :

  * einen Phenylkern, gegebenenfalls substituiert durch eine Gruppe Halogen, Alkoxy mit 1 bis 4 Kohlenstoffatomen, Cyano, Carbonylamino, gegebenenfalls substituiert durch eine oder mehrere Gruppen Alkyl mit 1 bis 4 Kohlenstoffatomen, Guanyl, Alkylthio mit 1 bis 4 Kohlenstoffatomen, Amino, Alkylamino mit 1 bis 4 Kohlenstoffatomen, Dialkylamino mit 1 bis 4 Kohlenstoffatomen für jede Gruppe Alkyl, Nitro, Alkylenamino mit 1 bis 4 Kohlenstoffatomen oder Alkenylenamino mit 2 bis 4 Kohlenstoffatomen oder
  * einen mono- oder bi- oder tricyclischen heterocyclischen Kern mit 0 bis 2 Heteroatomen pro Ring, unter der Bedingung, daß mindestens ein Heteroatom in mindestens einem Ring anwesend ist, gegebenenfalls substituiert durch eine oder mehrere Gruppen Alkyl mit 1 bis 4 Kohlenstoffatomen oder durch Gruppen Alkylen mit 1 bis 4 Kohlenstoffatomen oder Gruppen Alkenylen mit 2 bis 4 Kohlenstoffatomen,

oder eines ihrer Salze.

**5.** Verbindungen nach Anspruch 3, **dadurch gekennzeichnet, daß** $Ar_1$ und $Ar_2$ eine unter den folgenden Gruppen ausgewählte Gruppe darstellen : 4-Amino- oder 4-Methylamino- oder 4-Dimethylamino-chinolyl oder -chinolinium, dessen Chinolinium-Kern gegebenenfalls durch eine Gruppe Methyl substituiert ist.

**6.** Verbindungen nach Anspruch 1, **dadurch gekennzeichnet, daß** die Gruppe A den Rest Thiomethyl, Amino, Alkylamino oder Dialkylamino darstellt, worin die Gruppen Alkyl 1 bis 4 Kohlenstoffatome besitzen.

**7.** Verbindungen nach Anspruch 2, **dadurch gekennzeichnet, daß** A eine Gruppe Methylthio darstellt.

**8.** Verbindungen nach Anspruch 1, **dadurch gekennzeichnet, daß** sie eine Inhibitorwirkung von Telomerasen besitzen.

**9.** Verbindungen nach irgendeinem der vorstehenden Ansprüche, **dadurch gekennzeichnet, daß** sie eine Antikrebswirkung besitzen.

**10.** Neue Verbindungen der folgenden Formel (I)

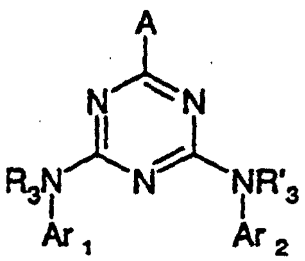

(I)

in der

- A darstellt :
    - eine Gruppe Amino der Formel $NR_1R_2$, worin $R_1$ und $R_2$, gleich oder verschieden, Wasserstoff oder eine gerade oder verzweigte Gruppe Alkyl mit 1 bis 4 Kohlenstoffatomen darstellen, oder
    - eine Gruppe $OR_1$ oder $SR_1$, worin $R_1$ Wasserstoff ist oder die gleiche Bedeutung wie vorstehend besitzt, oder
    - eine Gruppe Alkyl mit 1 bis 4 Kohlenstoffatomen oder eine Gruppe Trifluormethyl, oder
    - ein Wasserstoffatom oder
    - ein Halogenatom, ausgewählt unter Fluor, Chlor, Brom oder Iod,
- $R_3$ und $R'_3$, gleich oder verschieden, unabhängig voneinander ein Wasserstoffatom oder eine Gruppe Alkyl mit 1 bis 4 Kohlenstoffatomen darstellen,
- $Ar_1$ und $Ar_2$, gleich oder verschieden, darstellen :

    1. wenn $Ar_1$ und $Ar_2$ gleich sind :

        - eine Struktureinheit Chinolin, gegebenenfalls substituiert durch mindestens
            - eine Gruppe N(Ra)(Rb), worin Ra und Rb, gleich oder verschieden, Wasserstoff oder einen Rest Alkyl mit 1 bis 4 Kohlenstoffatomen darstellen, oder
            - eine Gruppe Alkoxy mit kurzer Kette von 1 bis 4 Kohlenstoffatomen, oder
        - ein Chinolin mit einem Stickstoffatom in quaternärer Form oder
        - ein Benzamidin, außer in dem Fall, wo A Diethylamin, Wasserstoff oder eine Gruppe Amin bedeutet,
        - ein Pyridin, gebunden in Position -4 oder verbunden mit einer Gruppe Aryl oder Heteroaryl, gegebenenfalls substituiert durch eine Gruppe Alkyl mit 1 bis 4 Kohlenstoffatomen,

    2. wenn $Ar_1$ und $Ar_2$ verschieden sind :

        - $Ar_1$ und $Ar_2$ beide eine der oben bei $Ar_1$ und $Ar_2$ erwähnten Möglichkeiten darstellen, oder
        - $Ar_1$ eine der obigen Möglichkeiten bedeutet und $Ar_2$ darstellt :
            * einen Phenylkern, gegebenenfalls substituiert durch eine Gruppe Halogen, Alkoxy mit 1 bis 4 Kohlenstoffatomen, Cyano, Carbonylamino, gegebenenfalls substituiert durch eine oder mehrere Gruppen Alkyl mit 1 bis 4 Kohlenstoffatomen, Guanyl, Alkylthio mit 1 bis 4 Kohlenstoffatomen, Amino, Alkylamino mit 1 bis 4 Kohlenstoffatomen, Dialkylamino mit 1 bis 4 Kohlenstoffatomen für jede Gruppe Alkyl, Nitro, Alkylenamino mit 1 bis 4 Kohlenstoffatomen oder Alkenylenamino mit 2 bis 4 Kohlenstoffatomen oder
            * einen mono- oder bi- oder tricyclischen heterocyclischen Kern mit 0 bis 2 Heteroatomen pro Ring, unter der Bedingung, daß mindestens ein Heteroatom in mindestens einem Ring anwesend ist, gegebenenfalls substituiert durch eine oder mehrere Gruppen Alkyl mit 1 bis 4 Kohlenstoffatomen oder durch Gruppen Alkylen mit 1 bis 4 Kohlenstoffatomen oder Gruppen Alkenylen mit 2 bis 4 Kohlenstoffatomen,

oder eines ihrer Salze, mit Ausschluß von 2-Amino-bis-4,6-[(4'-Amino-6'-chinaldinyl)-amino]-triazin-Dihydrochlorid und 2-Aminobis-4,6-(p-amidino-anilino)-triazin.

**11.** Verbindungen nach Anspruch 10, **dadurch gekennzeichnet, daß** in dem Fall, wo $Ar_1$ und $Ar_2$ gleich sind, $Ar_1$ und $Ar_2$ eine Gruppe darstellen, ausgewählt unter den Gruppen 4-Amino- oder 4-Methylamino- oder 4-Dimethylamino-chinolyl oder -chinolinium, dessen Chinolinium-Kern gegebenenfalls durch eine Gruppe Methyl substituiert ist.

**12.** Verbindungen nach Anspruch 10, **dadurch gekennzeichnet, daß** $R_1$ und $R_2$ Wasserstoff darstellen.

**13.** Verbindungen nach Anspruch 10, **dadurch gekennzeichnet, daß** A eine Gruppe Methylthio ist.

**14.** Verbindungen nach Anspruch 10, **dadurch gekennzeichnet, daß** in dem Fall, wo $Ar_1$ und $Ar_2$ verschieden sind,

1. $Ar_1$ darstellt :

• eine Struktureinheit Chinolin, substituiert durch mindestens

  - eine Gruppe N(Ra)(Rb), worin Ra und Rb, gleich oder verschieden, Wasserstoff oder einen Rest Alkyl mit 1 bis 4 Kohlenstoffatomen darstellen, oder
  - eine Gruppe Alkoxy mit kurzer Kette von 1 bis 4 Kohlenstoffatomen, oder

• ein Chinolin mit einem Stickstoffatom in quaternärer Form oder
• ein Benzamidin, außer in dem Fall, wo A Diethylamin, Wasserstoff oder eine Gruppe Amin bedeutet, oder
• ein Pyridin, gebunden in Position -4 oder verbunden mit einer Gruppe Aryl oder Heteroaryl,

2. $Ar_2$ darstellt :

* einen wie oben definierten Kern, aber verschieden, oder
* einen Phenylkern, gegebenenfalls substituiert durch eine Gruppe Halogen, Methoxy, Cyano, Carbonylamino, Guanyl, Methylthio, Amino, Methylamino, Dimethylamino, Morpholin, Alkylenamino mit 1 bis 4 Kohlenstoffatomen oder Alkenylenamino mit 2 bis 4 Kohlenstoffatomen,
* einen Kern Chinolin, Benzimidazol, Indol, Benzothiophen, Benzofuran, Benzothiazol, Benzoxazol, Carbazol, Chinazolin, Chinoxalin, gegebenenfalls substituiert durch eine oder mehrere Gruppen Alkyl mit 1 bis 4 Kohlenstoffatomen oder durch Gruppen Alkylen mit 1 bis 4 Kohlenstoffatomen oder Gruppen Alkenylen mit 2 bis 4 Kohlenstoffatomen,

oder eines ihrer Salze, mit Ausschluß von 2-Amino-bis-4,6-[(4'-Amino-6'-chinaldinyl)-amino]-triazin-Dihydrochlorid und 2-Aminobis-4,6-(p-amidino-anilino)-triazin.

**15.** Verbindungen nach Anspruch 10, ausgewählt unter:

- 2-Amino-bis-4,6-[(1'-methyl-4'-amino-6'-chinaldinio)-amino]-triazin-dichlorid,
- 2-Amino-bis-4,6-[(1'-ethyl-4'-amino-6'-chinaldinio)-amino]-triazin-dichlorid,
- 2-Dimethylamino-bis-4,6-[(1'-methyl-4'-amino-6'-chinaldinio)-amino]-triazin-dichlorid,
- 2-Methylamino-bis-4,6-[(4'-amino-6'-chinaldinyl)-amino]-triazin-trihydrochlorid,
- 2-Amino-bis-4,6-[(1'-methyl-6'-chinoleinio)-amino]-triazindichlorid,
- 2-Methylamino-bis-4,6-[(4'-methylamino-6'-chinaldinyl)-amino]-triazin-dichlorid-trihydrochlorid,
- 2-Amino-bis-4,6-[(9'-amino-10'-methyl-2'-acridinio)-amino]-triazin-dichlorid-hydrochlorid,
- 2-Methylthio-bis-4,6-[(1'-methyl-4'-amino-6'-chinaldinio)-amino]-triazin-dichlorid,
- 2-Chlor-bis-4,6-[(4'-dimethylamino-6'-chinaldinyl)-amino]-triazin-dihydrochlorid-dihydrat,
- 2-Methylthio-bis-4,6-[(4'-dimethylamino-6'-chinaldinyl)-amino]-triazin-hydrat,
- N,N'-(4-Amino-6-chinaldinyl)-harnstoff-dihydrochlorid,
- $N^1$,$N^5$-Bis-(7-Chlor-1-methyl-4-chinoleinio)-pentan-1,5-diamindiiodid,
- Bis-2,4-[(4'Amino-6'-chinaldinyl)-amino]-pyrimidin-trihydrochlorid-pentahydrat,
- 1,5-(4'Amino-6'-chinaldinyl)-biguanid-trihydrochlorid-dihydrat,
- 6-[4-(4-Amino-2-methyl-chinolin-6-yl-amino)-6-methylsulfanyl-[1,3,5]triazin-2-yl-amino]-2-methyl-chinolin-4-ol,
- N6-[4-(4-Dimethylamino-2-methyl-chinolin-6-yl-amino)-6-methylsulfanyl-[1,3,5]triazin-2-yl]-2-methyl-chinolin-4,6-diamin,
- N6-[4-(4-Amino-2-methyl-chinolin-6-yl-amino)-6-methylsulfanyl-[1,3,5]triazin-2-yl]-2-methyl-chinolin-4,6-diamin,
- N6-[4-(4-Methoxy-2-methyl-chinolin-6-yl-amino)-6-methylsulfanyl-[1,3,5]triazin-2-yl]-4-methoxy-2-methyl-chinolin-6-amin,
- N6-{[6- (4-Amino-chinaldin-6-yl-amino) -4-methylthio] -triazin-2-yl}-chinaldin-4,6-diamin,
- N6-{[6-(4-Dimethylamino-chinaldin-6-yl-amino)-4-methylthio]-triazin-2-yl}-chinaldin-4,6-diamin,
- N6-{[6-(Chinolyl-6-yl-amino)-4-diethylamino]-triazin-2-yl}chinaldin-4,6-diamin.

**16.** Verbindungen nach Anspruch 15, ausgewählt unter:

- 2-Methylthio-bis-4,6-[(4'-dimethylamino-6'-chinaldinyl)-amino]-triazin-hydrat,
- 2-Chlor-bis-4,6-[(4'-dimethylamino-6'-chinaldinyl)-amino]-triazin-dihydrochlorid-dihydrat,
- 6-[4-(4-Amino-2-methyl-chinolin-6-yl-amino)-6-methylsulfanyl-[1,3,5]triazin-2-yl-amino]-2-methyl-chinolin-4-ol,
- N6-[4-(4-Dimethylamino-2-methyl-chinolin-6-yl-amino)-6-methylsulfanyl-[1,3,5]triazin-2-yl]-2-methyl-chinolin-4,6-diamin,
- N6-[4-(4-Amino-2-methyl-chinolin-6-yl-amino)-6-methylsulfanyl-[1,3,5]triazin-2-yl]-2-methyl-chinolin-4,6-diamin,
- N6-[4-(4-Methoxy-2-methyl-chinolin-6-yl-amino)-6-methylsulfanyl-[1,3,5]triazin-2-yl]-4-methoxy-2-methyl-chinolin-6-amin,
- N6-{[6-(4-Amino-chinaldin-6-yl-amino)-4-methylthio]-triazin-2-yl}-chinaldin-4,6-diamin,
- N6-{[6-(4-Dimethylamino-chinaldin-6-yl-amino)-4-methylthio]-triazin-2-yl}-chinaldin-4,6-diamin,
- N6-{[6-(Chinolyl-6-yl-amino)-4-diethylamino]-triazin-2-yl}chinaldin-4,6-diamin.

**17.** Verwendung der Verbindungen von Anspruch 10 als pharmazeutisches Produkt für den Humangebrauch.

**18.** Therapeutische Assoziationen, bestehend aus einer Verbindung nach Anspruch 1 und einer anderen Antikrebsverbindung.

**19.** Assoziationen nach Anspruch 19, **dadurch gekennzeichnet, daß** die Antikrebsverbindung ausgewählt wird unter den Alkylierungsmitteln, den Platinderivaten, den antibiotischen Mitteln, den Antimikrotubuli-Mitteln, den Anthracyclinen, den Topoisomerasen der Gruppen I und II, den Fluoropyrimidinen, den Analogen von Cytidin, den Analogen von Adenosin, den Enzymen und verschiedenen Verbindungen wie L-Asparaginase, Hydroxyharnstoff, Transretinoesäure, Suramin, Irinotecan, Topotecan, Dexrazoxan, Amifostin, Herceptin sowie den oestrogenen und androgenen Hormonen und den antivasculären Mitteln.

**20.** Therapeutische Assoziation, bestehend aus einer Verbindung nach Anspruch 1 und Strahlen.

**21.** Assoziationen nach irgendeinem der Ansprüche 18 bis 20, **dadurch gekennzeichnet, daß** jede der Verbindungen oder der Behandlungen gleichzeitig, getrennt oder aufeinanderfolgend angewendet werden kann.

## Claims

**1.** Compounds which bind the G-quadruplex structure of telomers, **characterized in that** they correspond to the following general formula:

nitrogen-containing aromatic ring - $NR_3$ -

distribution agent - $NR'_3$ - aromatic ring

in which

- the nitrogen-containing aromatic ring represents:

  ◊ a quinoline optionally substituted with at least

- one group N(Ra)(Rb) in which Ra and Rb, which are identical or different, represent hydrogen or a C1-C4 alkyl radical or
- one short-chain C1-C4 alkoxy group or

◊ a quinoline possessing a nitrogen atom in quaternary form or
◊ a benzamidine or
◊ a pyridine

• the aromatic ring represents

◊ a quinoline optionally substituted with at least

- one group N(Ra)(Rb) in which Ra and Rb, which are identical or different, represent hydrogen or a C1-C4 alkyl radical or
- one short-chain C1-C4 alkoxy group or

◊ a quinoline possessing a nitrogen atom in quaternary form or
◊ a benzamidine or
◊ a pyridine or
◊ a phenyl ring optionally substituted with a halogen group, C1-C4 alkoxy group, cyano group, carbonylamino group optionally substituted with one or more C1-C4 alkyl groups, guanyl groups, C1-C4 alkylthio groups, amino groups, C1-C4 alkylamino groups, C1-C4 dialkylamino groups for each alkyl group, nitro group, C1-C4 alkyleneamino group or C2-C4 alkenyleneamino group or
◊ a mono- or bi- or tricyclic heterocyclic ring comprising 0 to 2 heteroatoms per ring provided that at least one heteroatom is present in at least one ring optionally substituted with one or more C1-C4 alkyl groups or with C1-C4 alkylene or C2-C4 alkenylene groups

• R3 and R'3, which are identical or different, represent independently of one another hydrogen or a C1-C4 alkyl radical
• the distribution agent represents:

◊ a triazine group optionally substituted with an alkyl radical having 1 to 4 carbon atoms, a thio, oxy or amino radical which are themselves optionally substituted with one or more short-chain alkyl chains containing 1 to 4 carbon atoms or a halogen atom or
◊ a carbonyl group or
◊ a group C(=NH)-NH-C(=NH) or
◊ an alkyldiyl group containing 3 to 7 carbon atoms or
◊ a diazine group optionally substituted with the same groups as the triazine

or one of its salts.

**2.** Compounds according to Claim 1, **characterized in that** the distribution agent is chosen from the triazine or diazine groups.

**3.** Compounds according to Claim 2, **characterized in that** the diazine groups are pyrimidines.

**4.** Compounds according to Claim 1, **characterized in that** they correspond to formula (I) below:

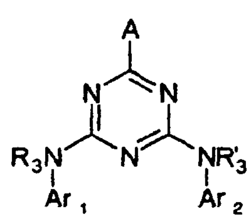

in which:

- A represents
    - an amino group of formula NR1R2 in which R1 and R2, which are identical or different, represent hydrogen or a straight or branched alkyl group containing 1 to 4 carbon atoms or
    - a group OR1 or SR1 in which R1 has the same meaning as above or
    - an alkyl group containing 1 to 4 carbon atoms or a trifluoromethyl group or
    - a hydrogen atom or
    - a halogen atom chosen from fluorine, chlorine, bromine or iodine
- R3 and R'3, which are identical or different, represent independently of one another hydrogen or a C1-C4 alkyl group
- $Ar_1$ and $Ar_2$, which are identical or different, represent

    1. when $Ar_1$ and $Ar_2$ are identical:

        - a quinoline motif optionally substituted with at least
            - one group N(Ra)(Rb) in which Ra and Rb, which are identical or different, represent hydrogen or a C1-C4 alkyl radical or
            - one short-chain alkoxy group containing 1 to 4 carbon atoms or
        - a quinoline possessing a nitrogen atom in quaternary form or
        - a benzamidine or
        - a pyridine attached at the 4-position or fused with an aryl or heteroaryl group optionally substituted with a C1-C4 alkyl group

    2. when $Ar_1$ and $Ar_2$ are different

        - $Ar_1$ and $Ar_2$ both represent one of the possibilities mentioned above for $Ar_1$ and $Ar_2$ or
        - $Ar_1$ represents one of the above possibilities and $Ar_2$ represents
            * a phenyl ring optionally substituted with a halogen group, C1-C4 alkoxy group, cyano group, carbonylamino group optionally substituted with one or more C1-C4 alkyl groups, guanyl groups, C1-C4 alkylthio groups, amino groups, C1-C4 alkylamino groups, C1-C4 dialkylamino groups for each alkyl group, nitro group, C1-C4 alkyleneamino group or C2-C4 alkenyleneamino group
            * a mono- or bi- or tricyclic heterocyclic ring comprising 0 to 2 heteroatoms per ring provided that at least one heteroatom is present in at least one ring optionally substituted with one or more C1-C4 alkyl groups or with C1-C4 alkylene or C2-C4 alkenylene groups

or one of its salts.

**5.** Compounds according to Claim 3, **characterized in that** $Ar_1$ and $Ar_2$ represent a group chosen from the following groups: 4-amino- or 4-methylamino- or 4-dimethylamino- quinolyl or -quinolinium in which the quinolinium ring is optionally substituted with a methyl group.

**6.** Compounds according to Claim 1, **characterized in that** the A group represents the methylthio, amino, alkylamino or dialkylamino radical, in which radicals the alkyl groups possess 1 to 4 carbon atoms.

**7.** Compounds according to Claim 2, **characterized in that** A represents a methylthio group.

**8.** Compounds of Claim 1, **characterized in that** they have a telomerase-inhibiting activity.

**9.** Compounds according to any one of the preceding claims, **characterized in that** they have an anticancer activity.

**10.** Novel compounds corresponding to the following formula (I) :

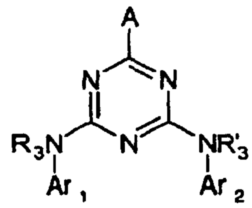

in which:

- A represents

  - an amino group of formula NR1R2 in which R1 and R2, which are identical or different, represent a straight or branched alkyl group containing 1 to 4 carbon atoms or
  - a group OR1 or SR1 in which R1 represents hydrogen or has the same meaning as above or
  - an alkyl group containing 1 to 4 carbon atoms or a trifluoromethyl group or
  - a hydrogen atom or
  - a halogen atom chosen from fluorine, chlorine, bromine or iodine

- R3 and R'3, which are identical or different, represent independently of one another a hydrogen atom or a C1-C4 alkyl group
- $Ar_1$ and $Ar_2$, which are identical or different, represent

  1. when $Ar_1$ and $Ar_2$ are identical:

  - a quinoline motif optionally substituted with at least

    - one group N(Ra) (Rb) in which Ra and Rb, which are identical or different, represent hydrogen or a C1-C4 alkyl radical or
    - one short-chain alkoxy group containing 1 to 4 carbon atoms or

  - a quinoline possessing a nitrogen atom in quaternary form or
  - a benzamidine except in the case where A represents diethylamine, hydrogen or an amine group
  - a pyridine attached at the 4-position or fused with an aryl or heteroaryl group optionally substituted with a C1-C4 alkyl group

  2. when $Ar_1$ and $Ar_2$ are different

  - $Ar_1$ and $Ar_2$ both represent one of the possibilities mentioned above for $Ar_1$ and $Ar_2$ or
  - $Ar_1$ represents one of the above possibilities and $Ar_2$ represents

    * a phenyl ring optionally substituted with a halogen group, C1-C4 alkoxy group, cyano group, carbonylamino group optionally substituted with one or more C1-C4 alkyl groups, guanyl groups, C1-C4 alkylthio groups, amino groups, C1-C4 alkylamino groups, C1-C4 dialkylamino groups for each alkyl group, nitro group, C1-C4 alkyleneamino group or C2-C4 alkenyleneamino group
    * a mono- or bi- or tricyclic heterocyclic ring comprising 0 to 2 heteroatoms per ring provided that at least one heteroatom is present in at least one ring optionally substituted with one or more C1-C4 alkyl groups or with C1-C4 alkylene or C2-C4 alkenylene groups

or one of its salts excluding 2-amino-bis-4,6-[(4'-amino-6'-quinaldinyl)amino]triazine dihydrochloride and 2-amino-bis-4,6-(p-amidino-anilino)tlriazine dihydrochloride.

**11.** Compounds according to Claim 10, **characterized in that** when $Ar_1$ and $Ar_2$ are identical, $Ar_1$ and $Ar_2$ represent a group chosen from 4-amino- or 4-methylanuno- or 4-dimethylamino- quinolyl or -quinolinium groups in which the quinolinium ring is optionally substituted with a methyl group.

**12.** Compounds according to Claim 10, **characterized in that** R1 and R2 represent hydrogen.

**13.** Compounds according to Claim 10, **characterized in that** A represents a methylthio group.

**14.** Compounds according to Claim 10, **characterized in that** when $Ar_1$ and $Ar_2$ are different

1. $Ar_1$ represents:

- a quinoline motif substituted with at least
    - one group N(Ra)(Rb) in which Ra and Rb, which are identical or different, represent hydrogen or a C1-C4 alkyl radical or
    - one short-chain alkoxy group containing 1 to 4 carbon atoms or
- a quinoline possessing a nitrogen atom in quaternary form or
- a benzamidine except in the case where A represents diethylamine, hydrogen or an amine group or
- a pyridine attached at the 4-position or fused with an aryl or heteroaryl group

2. $Ar_2$ represents

* a ring as defined above but different or
* a phenyl ring optionally substituted with a halogen, methoxy, cyano, carbonylamino, guanyl, methylthio, amino, methylamino, dimethylamino, morpholine, C1-C4 alkyleneamino or C2-C4 alkenyleneamino group
* a quinoline, benzimidazole, indole, benzothiophene, benzofuran, benzothiazole, benzoxazole, carbazole, quinazoline or quinoxaline ring optionally substituted with one or more C1-C4 alkyl groups or with C1-C4 alkylene or C2-C4 alkenylene groups

or one of its salts excluding 2-amino-bis-4,6-[(4'-amino-6'-quinaldinyl)amino]triazine dihydrochloride and 2-amino-bis-4,6-(p-amidinoanilino)triazine dihydrochloride.

**15.** Compounds according to Claim 10 chosen from:

- 2-amino-bis-4,6-[(1'-methyl-4'-amino-6'-quinaldinio)amino]triazine dichloride
- 2-amino-bis-4,6-[(1'-ethyl-4'-amino-6'-quinaldinio)amino]triazine dichloride
- 2-dimethylamino-bis-4,6-[(1'-methyl-4'-amino-6'-quinaldinio)amino]triazine dichloride
- 2-methylamino-bis-4,6-[(4'-amino-6'-quinaldinyl)amino]triazine trihydrochloride
- 2-amino-bis-4,6-[(1'-methyl-6'-quinolinio)-amino]triazine dichloride
- 2-methylamino-bis-4,6-[(4'-methylamino-6'-quinaldinyl)amino]triazine dichloride trihydrochloride
- 2-amino-bis-4,6-[(9'-amino-10'-methyl-2'-acridinio)amino]triazine dichloride hydrochloride
- 2-methylthio-bis-4,6-[(1'-methyl-4'-amino-6'-quinaldinio)amino]triazine dichloride
- 2-chloro-bis-4,6-[(4'-dimethylamino-6'-quinaldinyl)amino]triazine dihydrochloride dihydrate
- 2-methylthio-bis-4,6-[(4'-dimethylamino-6'-quinaldinyl)amino]triazine hydrate
- N,N'-(4-amino-6-quinaldinyl)urea dihydrochloride
- $N^1$,$N^5$-bis(7-chloro-1-methyl-4-quinolinio)-pentane-1,5-diamine diiodide
- bis-2,4-[(4'-amino-6'-quinaldinyl)amino]-pyrimidine trihydrochloride pentahydrate
- 1,5-(4'-amino-6'-quinaldinyl)biguanide trihydrochloride dihydrate
- 6-[4-(4-amino-2-methylquinolin-6-ylamino)-6-methylsulphanyl-[1,3,5]triazin-2-ylamino]-2-methyl-quinolin-4-ol
- N6-[4-(4-dimethylamino-2-methylquinolin-6-ylamino)-6-methylsulphanyl-[1,3,5]triazin-2-yl]-2-methylquinolin-4,6-diamine
- N6-[4-(4-amino-2-methylquinolin-6-ylamino)-6-methylsulphanyl-[1,3,5]triazin-2-yl]-2-methylquinolin-4,6-diamine
- N6-[4-(4-methoxy-2-methylquinolin-6-ylamino)-6-methylsulphanyl-[1,3,5]triazin-2-yl]-4-methoxy-2-methylquinolin-6-amine
- N6-[6-(4-aminoquinaldin-6-ylamino)-4-methylthiotriazin-2-yl]quinaldine-4,6-diamine
- N6-[6-(4-dimethylaminoquinaldin-6-ylamino)-4-methylthiotriazin-2-yl]quinaldine-4,6-diamine
- N6-[6-(quinolyl-6-ylamino)-4-diethylamino-triazin-2-yl]quinaldine-4,6-diamine.

**16.** Compounds according to Claim 15 chosen from:

- 2-methylthio-bis-4,6-[(4'-dimethylamino-6'-quinaldinyl)amino]triazine hydrate
- 2-chloro-bis-4,6-[(4'-dimethylamino-6'-quinaldinyl)amino]triazine dihydrochloride dihydrate
- 6-[4-(4-amino-2-methylquinolin-6-ylamino)-6-methylsulphinyl-[1,3,5]triazin-2-ylamino]-2-methyl-quinolin-4-ol
- N6-[4-(4-dimethylamino-2-methylquinolin-6-ylamino)-6-methylsulphanyl-[1,3,5]triazin-2-yl]-2-methylquinoline-4,6-diamine
- N6-[4-(4-amino-2-methylquinolin-6-ylamino)-6-methylsulphanyl-[1,3,5]triazin-2-yl]-2-methyl-quinoline-4,6-diamine
- N6-[4-(4-methoxy-2-methylquinolin-6-ylamino)-6-methylsulphanyl-[1,3,5]triazin-2-yl]-4-methoxy-2-methylquinolin-6-amine
- N6-[6-(4-aminoquinaldin-6-ylamino)-4-methyl-thiotriazin-2-yl]quinaldine-4,6-diamine
- N6-[6-(4-dimethylaminoquinaldin-6-ylamino)-4-methylthiotriazin-2-yl]quinaldine-4,6-diamine
- N6-[6-(quinolyl-6-ylamino)-4-diethyl-aminotriazin-2-yl]quinaldine-4,6-diamine.

**17.** Use of the compounds of Claim 10 as pharmaceutical product for human use.

**18.** Therapeutic combinations consisting of a compound according to Claim 1 and of another anticancer compound.

**19.** Combinations according to Claim 18, **characterized in that** the anticancer compound is chosen from alkylating agents, platinum derivatives, antibiotic agents, antimicrotubule agents, anthracyclines, group I and II topoisomerases, fluoropyrimidines, cytidine analogues, adenosine analogues, various enzymes and compounds such as L-asparaginase, hydroxyurea, trans-retinoic acid, suramine, irinotecan, topotecan, dexrazoxane, amifostine, herceptin as well as oestrogenic and androgenic hormones, antivascular agents.

**20.** Therapeutic combination consisting of a compound according to Claim 1 and of radiation.

**21.** Combinations according to any one of Claims 18 to 20, **characterized in that** each of the compounds or treatments is administered simultaneously, separately or sequentially.